# EUROPEAN PATENT APPLICATION

(11) **EP 0 887 341 A1**
(43) Date of publication of application: **30.12.1998**
(21) Application number: 98111505.8
(22) Date of filing: 23.06.1998
(51) Int. Cl.: C07C 237/42, A61K 31/16, C07D 209/18, C07D 333/68, C07D 317/68, C07D 307/84

(54) **Amide derivatives**

(30) Priority: 25.06.1997 JP 168484/97
(71) Applicant: MITSUBISHI CHEMICAL CORPORATION, Chiyoda-ku, Tokyo 100-0005 (JP)
(72) Inventor: Ando, Ryoichi, Mitsubishi Chemical Corporation, Yokohama-shi, Kanagawa 227-8502 (JP); Kawamura, Makoto, Tokyo 144-0051 (JP); Chiba, Noriko, Mitsubishi Chemical Corporation, Yokohama-shi, Kanagawa 227-8502 (JP); Watanabe, Kazutoshi, Mitsubishi Chemical Corp., Yokohama-shi, Kanagawa 227-8502 (JP)
(74) Representative: Godemeyer, Thomas, Dr.

(57) **Abstract**

A compound represented by the following general formula (I): 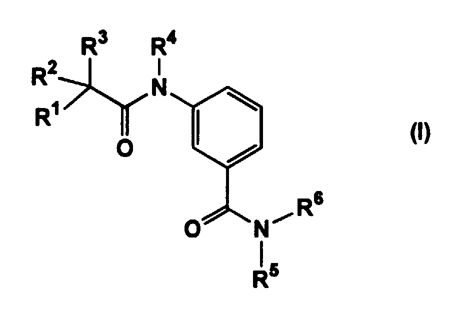 wherein R¹ represents a C₃-C₈ cycloalkyl group, an optionally substituted C₆-C₁₄ aryl group, an optionally substituted heterocyclic residue, an optionally substituted C₆-C₁₄ aryloxy group, or an optionally substituted C₇-C₁₅ arylmethyl group; R² and R³ independently represent hydrogen atom or a C₁-C₅ alkyl group, or R² and R³ may combine to represent a C₂-C₇ alkylene group; R⁴ and R⁵ independently represent hydrogen atom or a C₁-C₅ alkyl group; and R⁶ represents hydrogen atom, a C₁-C₅ alkyl group which may optionally be substituted with a hydroxyl group, hydroxyl group or a C₁-C₅ alkoxy group, or a salt thereof, or a solvate thereof or a hydrate thereof. The compounds have antibacterial activity against Helicobacter pylori.

## Description

### Technical Field

The present invention relates to novel amide derivatives having strong antibacterial activity against Helicobacter pylori.

### Background Art

Helicobacter pylori is a slightly aerobic gram-negative bacterium which was recently isolated from human gastric mucosa, and various published reports suggest its involvement in inflammation of alimentary tract, formation and recurrence of ulcer, and moreover, gastric cancer (Molecular Medicine, Vol. 31, pp.1304-1374, 1994).

For the treatment of gastrointestinal ulcers, medicaments such as H₂ blockers or proton pump inhibitors have been used so far. Since relation between Helicobacter pylori infection and gastric ulcer has been being clarified as explained above, an antibacterial agent such as amoxicillin has become practically used in combination, particularly from a viewpoint of prevention of recurrence. However, in most cases, ordinarily used antibacterial agents fail to achieve complete elimination of the bacteria. In addition, they may affect on intestinal bacterial flora due to their broad antibacterial spectra, and they often cause adverse effects such as diarrhea. Therefore, it has been desired to develop an antibacterial agent having potent antibacterial activity in alimentary tract that is specific against Helicobacter pylori.

### Disclosure of the Invention

The inventors of the present invention conducted researches to provide a novel anti-Helicobacter pylori agent, and as a result, they found that the compounds represented by the following general formula have excellent antibacterial activity against Helicobacter pylori, and can exhibit potent antibacterial activity in alimentary tract. The present invention was achieved on the basis of these findings.

The present invention thus provides amide derivatives represented by the following general formula (I) and salts thereof and solvates thereof and hydrates thereof: 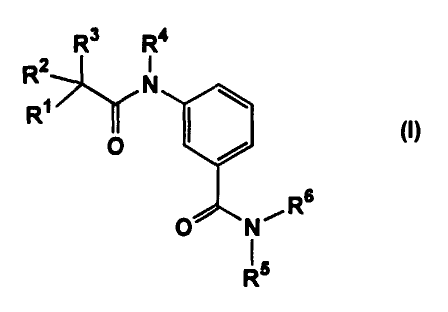 wherein R¹ represents a C₃-C₈ cycloalkyl group, a C₆-C₁₄ aryl group which may optionally be substituted, a residue of a heterocyclic compound which may optionally be substituted, a C₆-C₁₄ aryloxy group which may optionally be substituted, or a C₇-C₁₅ arylmethyl group which may optionally be substituted; R² and R³ independently represent hydrogen atom or a C₁-C₅ alkyl group, or R² and R³ may combine together to represent a C₂-C₇ alkylene group; R⁴ and R⁵ independently represent hydrogen atom or a C₁-C₅ alkyl group; and R⁶ represents hydrogen atom, a C₁-C₅ alkyl group which may optionally be substituted with a hydroxyl group, hydroxyl group, or a C₁-C₅ alkoxy group.

According to another aspect of the present invention, the present invention provides medicaments, preferably for the treatment of gastric diseases, e.g., gastritis, gastric ulcer, and gastric cancer, which comprise as an active ingredient a substance selected from the group consisting of the aforementioned amide derivatives and pharmaceutically acceptable salts thereof and solvates thereof and hydrates thereof. The medicaments are preferably provided as pharmaceutical compositions comprising the aforementioned substance as an active ingredient together with one or more pharmaceutically acceptable additives. These medicaments can be used as anti-Helicobacter pylori agents for therapeutic and/or preventive treatment of digestive diseases related to Helicobacter pylori infection, for example, gastritis, gastric ulcer, gastric cancer, stomach malignant lymphoma, MALT lymphoma, duodenal ulcer, duodenal carcinoma and the like.

According to further aspects of the present invention, there are provided a method for treating digestive diseases related to Helicobacter pylori infection which comprises the step of administering to a mammal including a human a therapeutically effective amount of a substance selected from the group consisting of the aforementioned amide derivatives and pharmaceutically acceptable salts thereof and solvates thereof and hydrates thereof; and a use of a substance selected from the group consisting of the aforementioned amide derivatives and pharmaceutically acceptable salts thereof, and solvates thereof and hydrates thereof for the manufacture of the above medicaments.

### Best Mode for Carrying Out the Invention

In the general formula (I), examples of the C₃-C₈ cycloalkyl group represented by R¹ include, for example, cyclopropyl group, cyclobutyl group, cyclopentyl group, cyclohexyl group, cycloheptyl group, and cyclooctyl group. Examples of the C₆-C₁₄ aryl group include, for example, aromatic hydrocarbon groups consisting of one ring or two to approximately three condensed aromatic rings such as phenyl group, naphthyl group, and anthryl group.

As the residue of a heterocyclic compound, residues of heterocyclic compounds containing 1 to 4 heteroatoms selected from oxygen atom, sulfur atom, and nitrogen atom, and having 5 to 10 ring-membered atoms in total can be used. More specifically, examples of the residues of heterocyclic compounds include, for example, furan ring, dihydrofuran ring, tetrahydrofuran ring, pyran ring, dihydropyran ring, tetrahydropyran ring, benzofuran ring, dihydrobenzofuran ring, isobenzofuran ring, chromene ring, chroman ring, isochroman ring, thiophene ring, benzothiophene ring, pyrrole ring, pyrroline ring, pyrrolidine ring, imidazole ring, imidazoline ring, imidazolidine ring, pyrazole ring, pyrazoline ring, pyrazolidine ring, triazole ring, tetrazole ring, pyridine ring, pyridineoxide ring, piperidine ring, pyrazine ring, piperazine ring, pyrimidine ring, pyridazine ring, indolizine ring, indole ring, indoline ring, isoindole ring, isoindoline ring, indazole ring, benzimidazole ring, purine ring, quinolizine ring, quinoline ring, phthalazine ring, naphthylidine ring, quinoxaline ring, quinazoline ring, cinnoline ring, pteridine ring, oxazole ring, oxazolidine ring, isoxazole ring, isoxazolidine ring, thiazole ring, thiazylidine ring, isothiazole ring, isothiazolidine ring, dioxane ring, dithian ring, morpholine ring, and thiomorpholine ring. Examples of the C₆-C₁₄ aryloxy group include, for example, phenyloxy group, naphthyloxy group, and anthryloxy group, and examples of the C₇-C₁₅ arylmethyl group include, for example, benzyl group, naphthylmethyl group, and anthrylmethyl group.

The C₁-C₅ alkyl group independently represented by R² and R³ may be either a straight-or branched-chain alkyl, and examples include, for example, methyl group, ethyl group, propyl group, isopropyl group, butyl group, isobutyl group, pentyl group, and isopentyl group. Examples of the C₂-C₇ alkylene group represented by R² combined with R³ include, for example, ethylene group, trimethylene group, tetramethylene group, pentamethylene group, hexamethylene group, and heptamethylene group, and these groups may have one or more branched chains.

As the C₁-C₅ alkyl group represented by R⁴ and R⁵, those explained for R² and R³ can be independently used.

As the C₁-C₅ alkyl group represented by R⁶, those explained for R² and R³ can be used, and these alkyl groups may be substituted with at least one, preferably one hydroxy group. The C₁-C₅ alkoxy group represented by R⁶ may be a straight or branched chain group, and examples include, for example, methoxy group, ethoxy group, propoxy group, isopropoxy group, butoxy group, isobutoxy group, tert-butoxy group, pentyloxy group, and isopentyloxy group.

The aforementioned aryl group, residue of a heterocyclic compound, aryloxy group, and arylmethyl group may have one or more substituents at arbitrary positions on their rings. Examples of substituents include, for example, a halogen atom such as fluorine atom, chlorine atom, and bromine atom; a C₁-C₅ alkyl group such as methyl group, ethyl group, propyl group, isopropyl group, butyl group, isobutyl group, sec-butyl group, tert-butyl group, pentyl group, isopentyl group, neopentyl group, and tert-pentyl group; a C₇-C₁₅ aralkyl group such as benzyl group, phenylethyl group, and naphthylmethyl group; trifluoromethyl group; a C₁-C₅ alkoxy group such as methoxy group, ethoxy group, propoxy group, isopropoxy group, butoxy group, isobutoxy group, tert-butoxy group, pentyloxy group, ad isopentyloxy group; a C₇-C₁₅ aralkyloxy group such as benzyloxy group, phenylethyloxy group, and naphthylmethyloxy group; a C₁-C₅ alkylenedioxy group such as methylenedioxy group, ethylenedioxy group, and propylenedioxy group; hydroxy group; nitro group; a C₂-C₆ alkylcarbonyloxy group such as acetoxy group, propionyloxy group, butyryloxy group, and valeryloxy group; carboxyl group; a C₂-C₆ alkoxycarbonyl group such as methoxycarbonyl group, ethoxycarbonyl group, propoxycarbonyl group, isopropoxycarbonyl group, butoxycarbonyl group, isobutoxycarbonyl group, tert-butoxycarbonyl group, and pentyloxycarbonyl group; a C₇-C₁₅ aralkyloxycarbonyl group such as benzyloxycarbonyl group, phenylethyloxycarbonyl group, and naphthylmethyloxycarbonyl group; oxo group; a C₂-C₆ alkylcarbonyl group such as acetyl group, propionyl group, butyryl group, and valeryl group; amino group; a C₁-C₅ monoalkylamino group such as methylamino group, ethylamino group, propylamino group, isopropylamino group, butylamino group, isobutylamino group, tert-butylamino group, pentylamino group, and isopentylamino group; a C₂-C₁₀ dialkylamino group such as dimethylamino group, ethylmethylamino group, diethylamino group, methylpropylamino group, and diisopropylamino group; a C₂-C₆ alkylcarbonylamino group such as acetylamino group, propionylamino group, isopropionylamino group, butyrylamino group, and valerylamino group; a C₂-C₆ alkoxycarbonylamino group such as methoxycarbonylamino group, ethoxycarbonylamino group, propoxycarbonylamino group, isopropoxycarbonylamino group, butoxycarbonylamino group, isobutoxycarbonylamino group, tert-butoxycarbonylamino group, and pentyloxycarbonylamino group; a C₇-C₁₅ aralkyloxycarbonylamino group such as benzyloxycarbonylamino group, phenylethyloxycarbonylamino group, and naphthylmethyloxycarbonylamino group; carbamoyl group; a C₂-C₆ alkylcarbamoyl group such as methylcarbamoyl group, ethylcarbamoyl group, propylcarbamoyl group, butylcarbamoyl group, tert-butylcarbamoyl group, and pentylcarbamoyl group; a C₆-C₁₂ aryl group such a phenyl group, and naphthyl group and the like.

Among the compounds of the present invention represented by the above formula (I), preferred compounds include those wherein R¹ is a C₆-C₁₄ aryl group which may optionally be substituted, a residue of a heterocyclic compound which may optionally be substituted, a C₆-C₁₄ aryloxy group which may optionally be substituted, or a C₇-C₁₅ arylmethyl group which may optionally be substituted, R², R³, R⁴ and R⁵ are hydrogen atoms, and R⁶ is a C₁-C₅ alkyl group. More preferred compounds include those wherein R¹ is a C₆-C₁₄ aryl group which may optionally be substituted, a residue of a heterocyclic compound which may optionally be substituted, a C₆-C₁₄ aryloxy group which may optionally be substituted, or a C₇-C₁₅ arylmethyl group which may optionally be substituted, R², R³, R⁴ and R⁵ are hydrogen atoms, and R⁶ is methyl group.

Examples of particularly preferred compounds include:
N-(3-methylcarbamoylphenyl)-3-chlorophenylacetamide;
N-(3-methylcarbamoylphenyl)-4-chlorophenylacetamide;
N-(3-methylcarbamoylphenyl)-3-bromophenylacetamide;
N-(3-methylcarbamoylphenyl)-4-bromophenylacetamide;
N-(3-methylcarbamoylphenyl)-3-methylphenylacetamide;
N-(3-methylcarbamoylphenyl)-4-methylphenylacetamide;
N-(3-methylcarbamoylphenyl)-3-methoxyphenylacetamide;
N-(3-methylcarbamoylphenyl)-4-methoxyphenylacetamide;
N-(3-methylcarbamoylphenyl)-3,4,5-trimethoxyphenylacetamide;
N-(3-methylcarbamoylphenyl)-3-benzyloxyphenylacetamide;
N-(3-methylcarbamoylphenyl)-1-naphthylacetamide;
N-(3-methylcarbamoylphenyl)-2-naphthylacetamide;
N-(3-methylcarbamoylphenyl)-3-indolylacetamide;
N-(3-methylcarbamoylphenyl)-3-benzothienylacetamide;
N-(3-methylcarbamoylphenyl)-4-benzothienylacetamide;
N-(3-methylcarbamoylphenyl)-3,4-methylenedioxyphenylacetamide;
N-(3-methylcarbamoylphenyl)-2-chlorophenoxyacetamide;
N-(3-methylcarbamoylphenyl)-2,3-dichlorophenoxyacetamide;
N-(3-methylcarbamoylphenyl)-1-naphthyloxyacetamide;
N-(3-methylcarbamoylphenyl)-2-naphthyloxyacetamide; and
N-(3-methylcarbamoylphenyl)-3-(2-methoxyphenyl)propionamide.

The amide derivatives of the present invention represented by the above general formula (I) can form a salt. Where one or more acidic groups exist, examples of salts include, for example, metal salts such as lithium salt, sodium salt, potassium salt, magnesium salt, and calcium salt, and ammonium salts such as inorganic ammonium salt, methylammonium salt, dimethylammonium salt, trimethylammonium salt, and dicyclohexylammonium salt. Where one or more basic groups exist, examples of salts include, for example, mineral acid salts such as hydrochloride, hydrobromide, sulfate, nitrate, and phosphate, and organic acid salts such as methanesulfonate, benzenesulonate, p-toluenesulfonate, acetate, propionate, tartrate, fumarate, maleate, malate, oxalate, succinate, citrate, benzoate, mandelate, cinnamate, and lactate. Pharmaceutically acceptable salts are preferred as the active ingredient of the medicaments of the present invention,. The amide derivatives of the present invention represented by the above general formula (I) and salts thereof may also exist as solvates or hydrates. Any substances in the form of salts, solvates, or hydrates as well as compounds in free forms fall within the scope of the present invention.

As to the stereochemistry of asymmetric carbon atoms present in the amide derivatives of the present invention represented by the general formula (I), the atoms can independently be in (S), (R), or (RS) configuration. Isomers in pure forms based on one or more asymmetric carbon atoms, e.g., enantiomers and diastereoisomers, any mixtures of such isomers, racemates and the like fall within the scope of the present invention.

Examples of specific examples of the amide derivatives of the present invention represented by the above general formula (I) include those listed in Table 1.

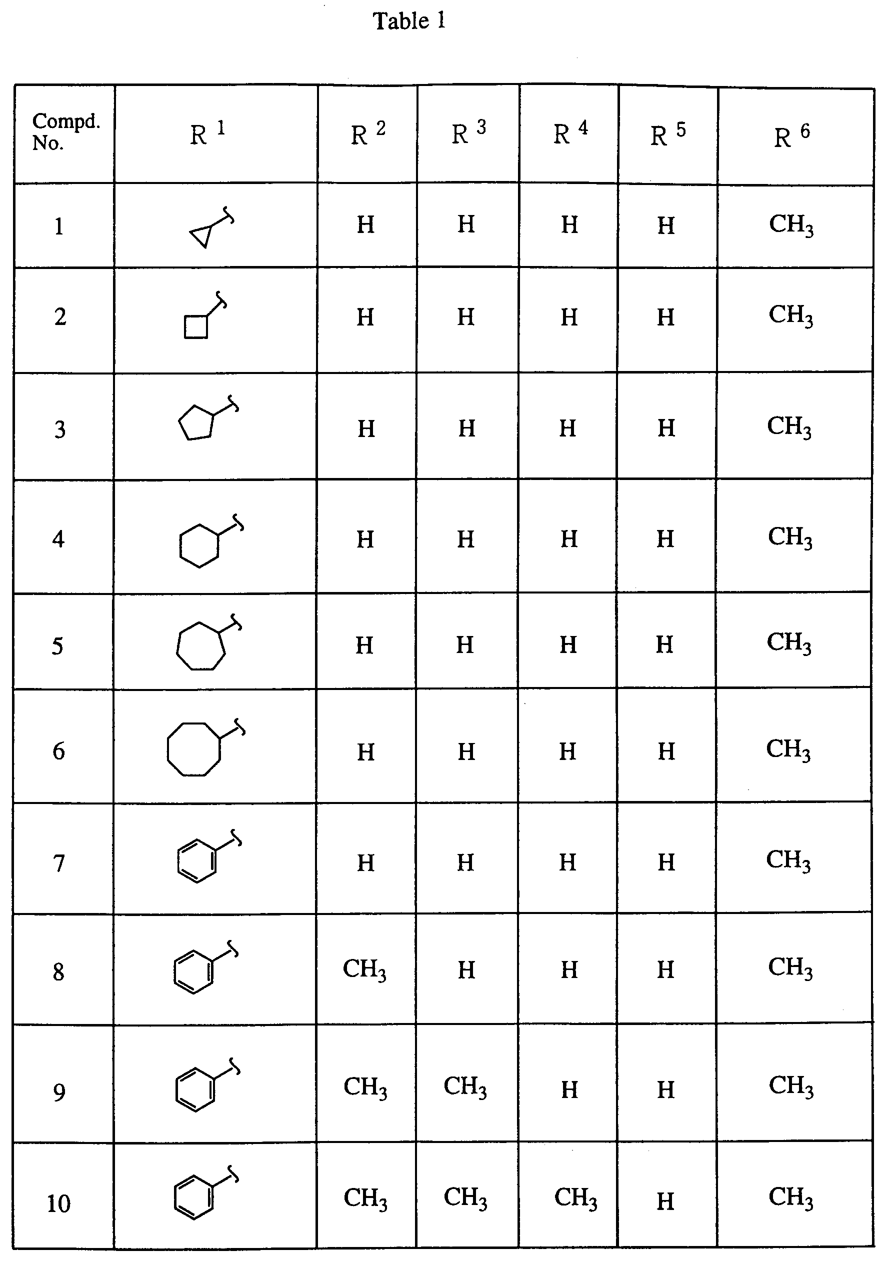

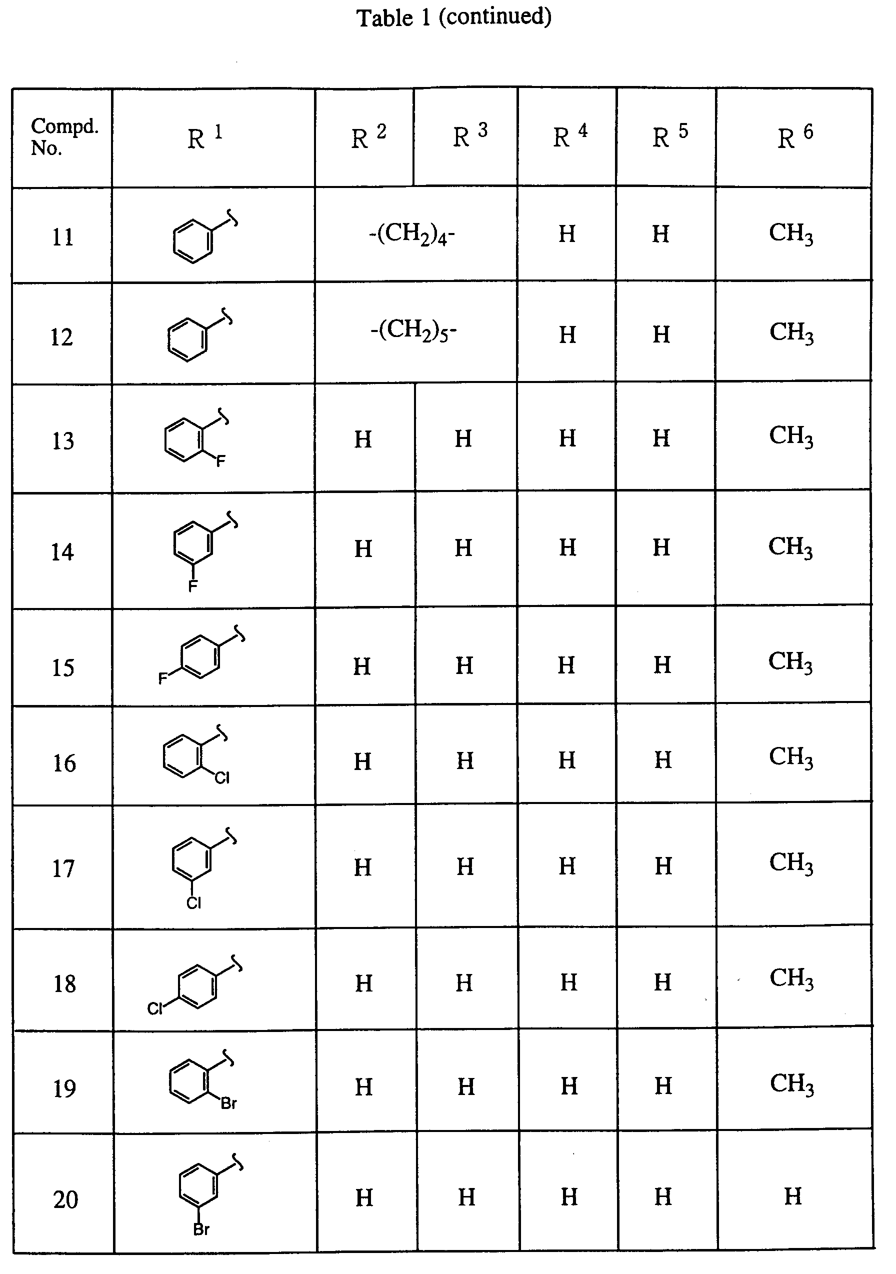

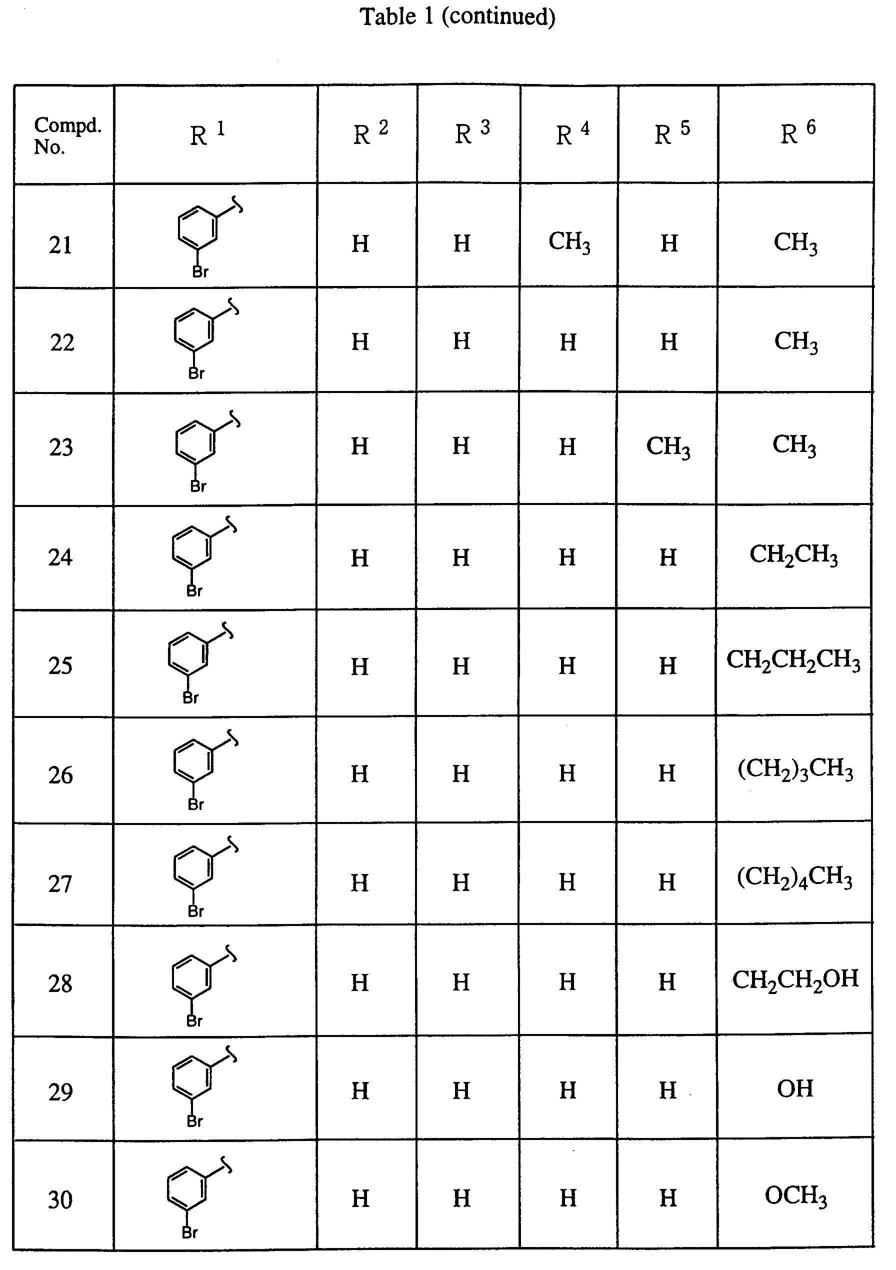

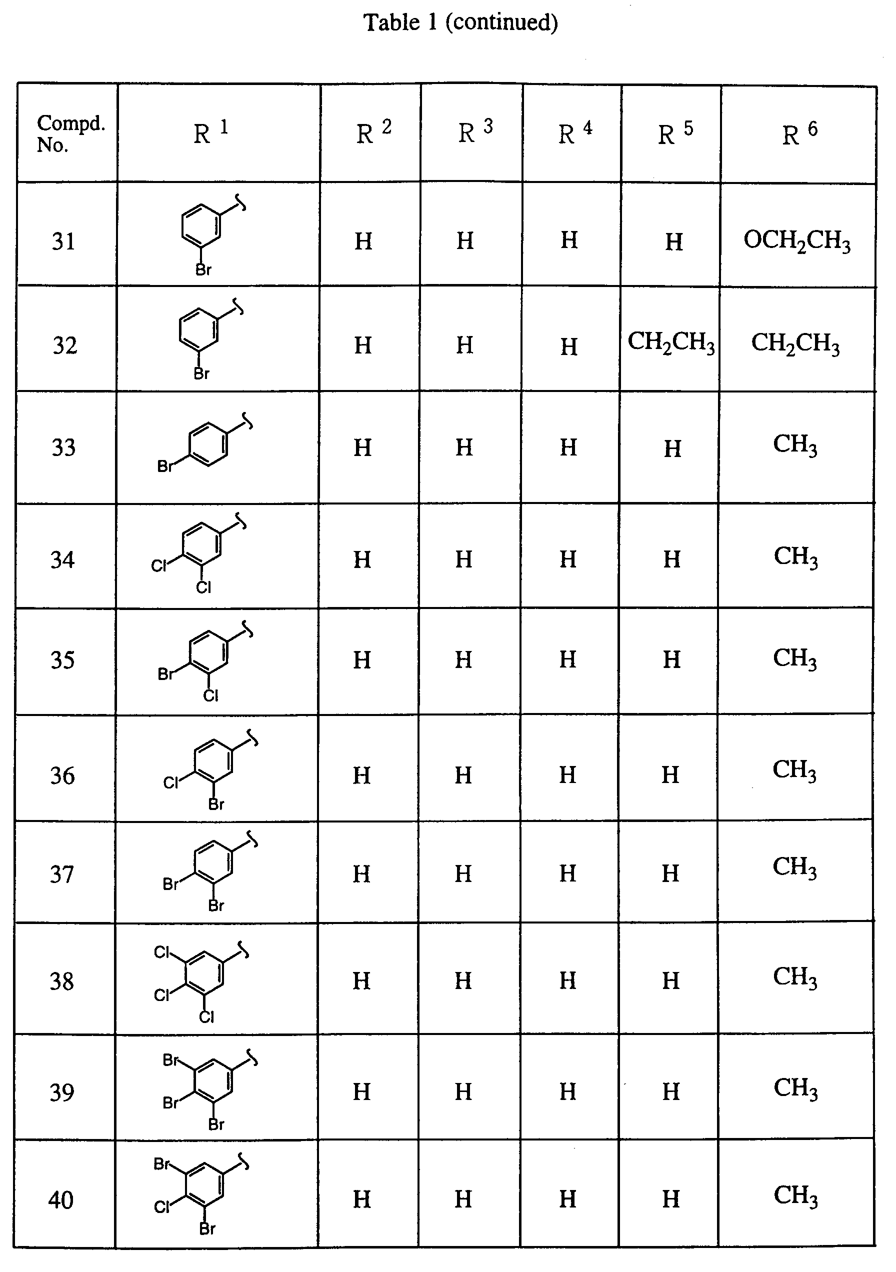

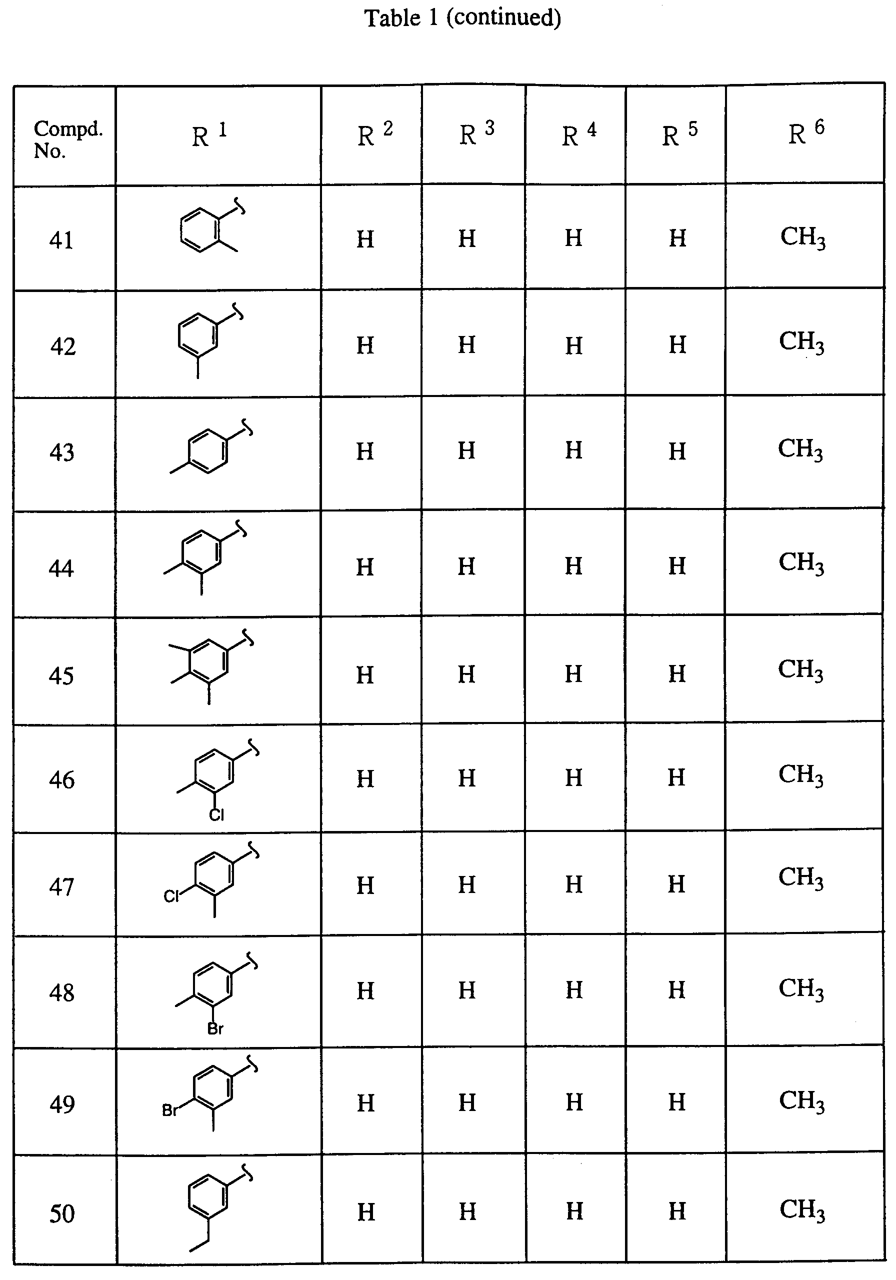

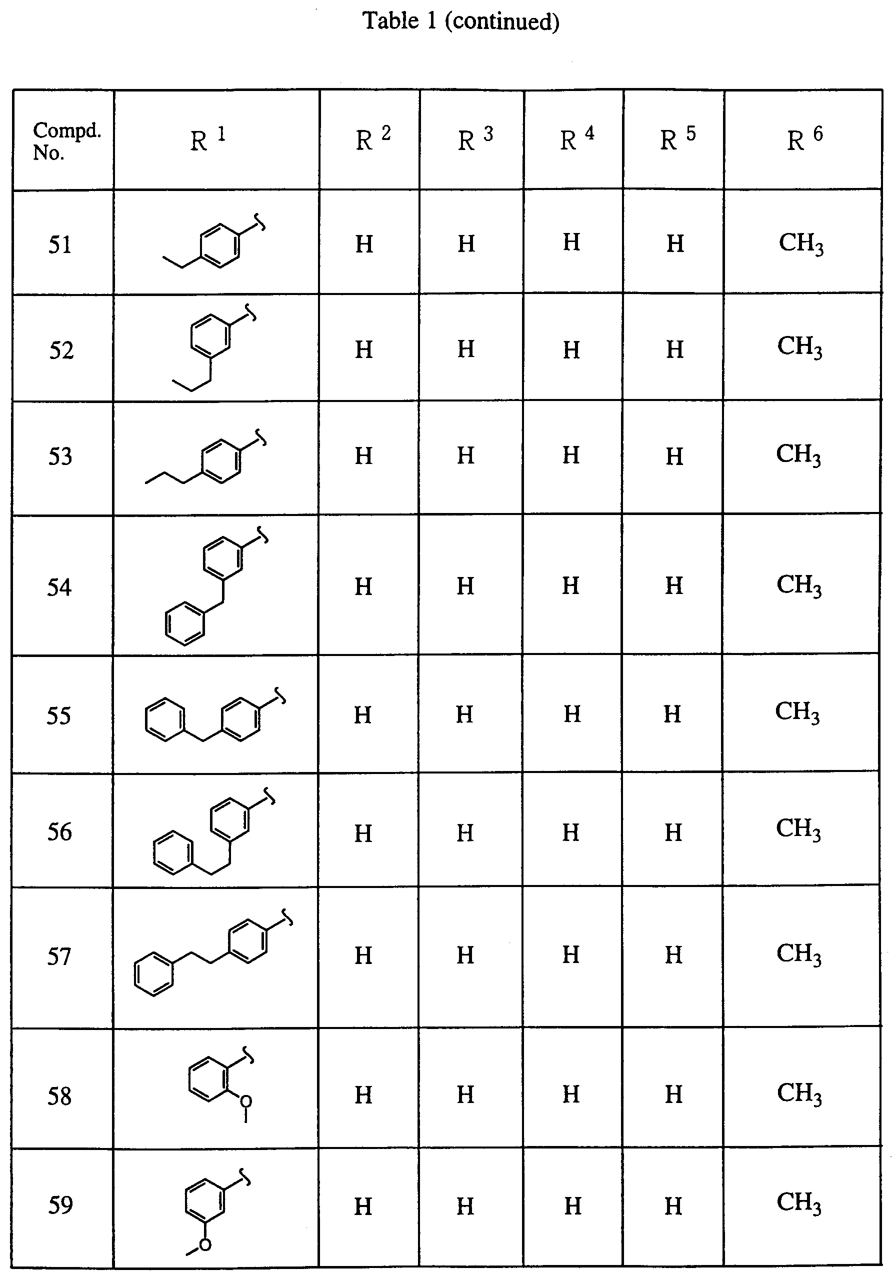

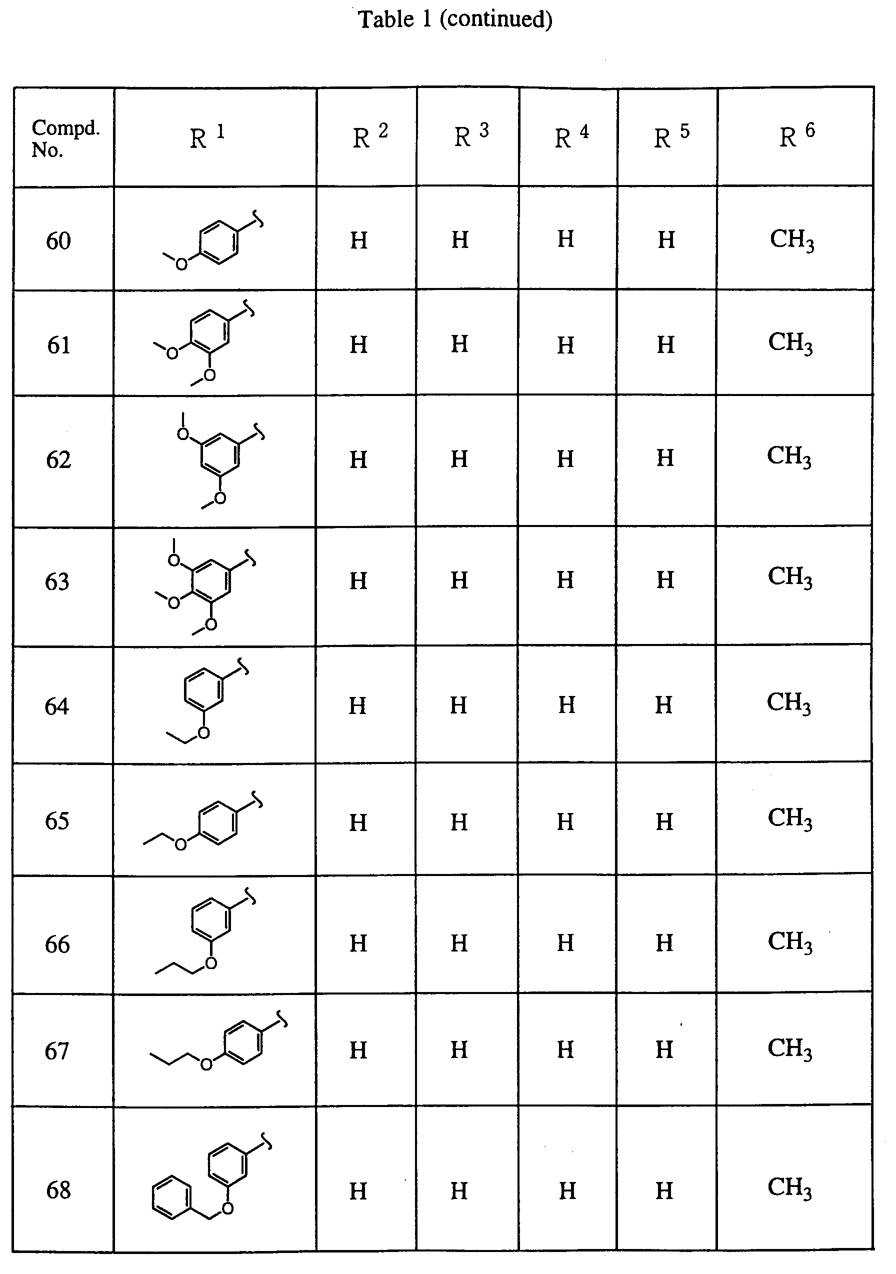

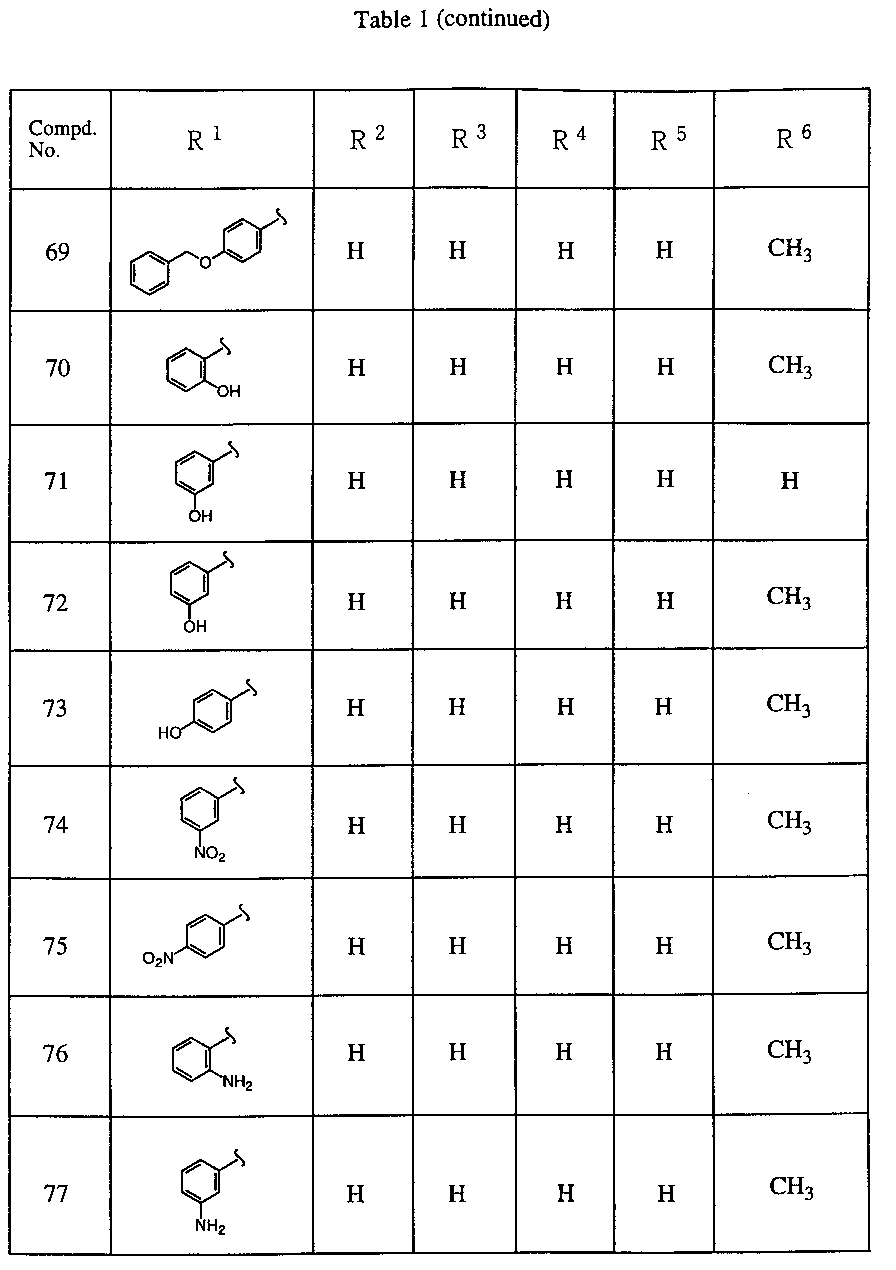

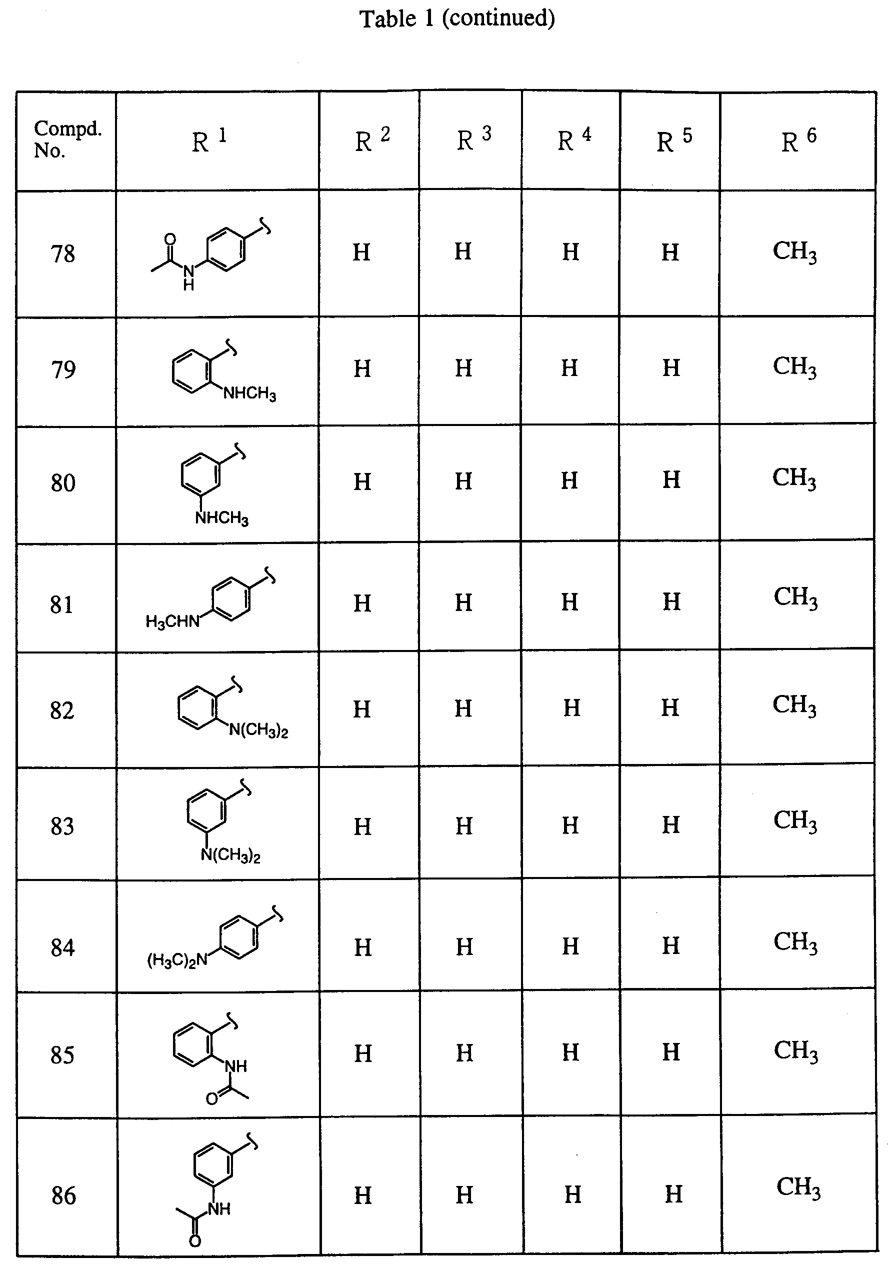

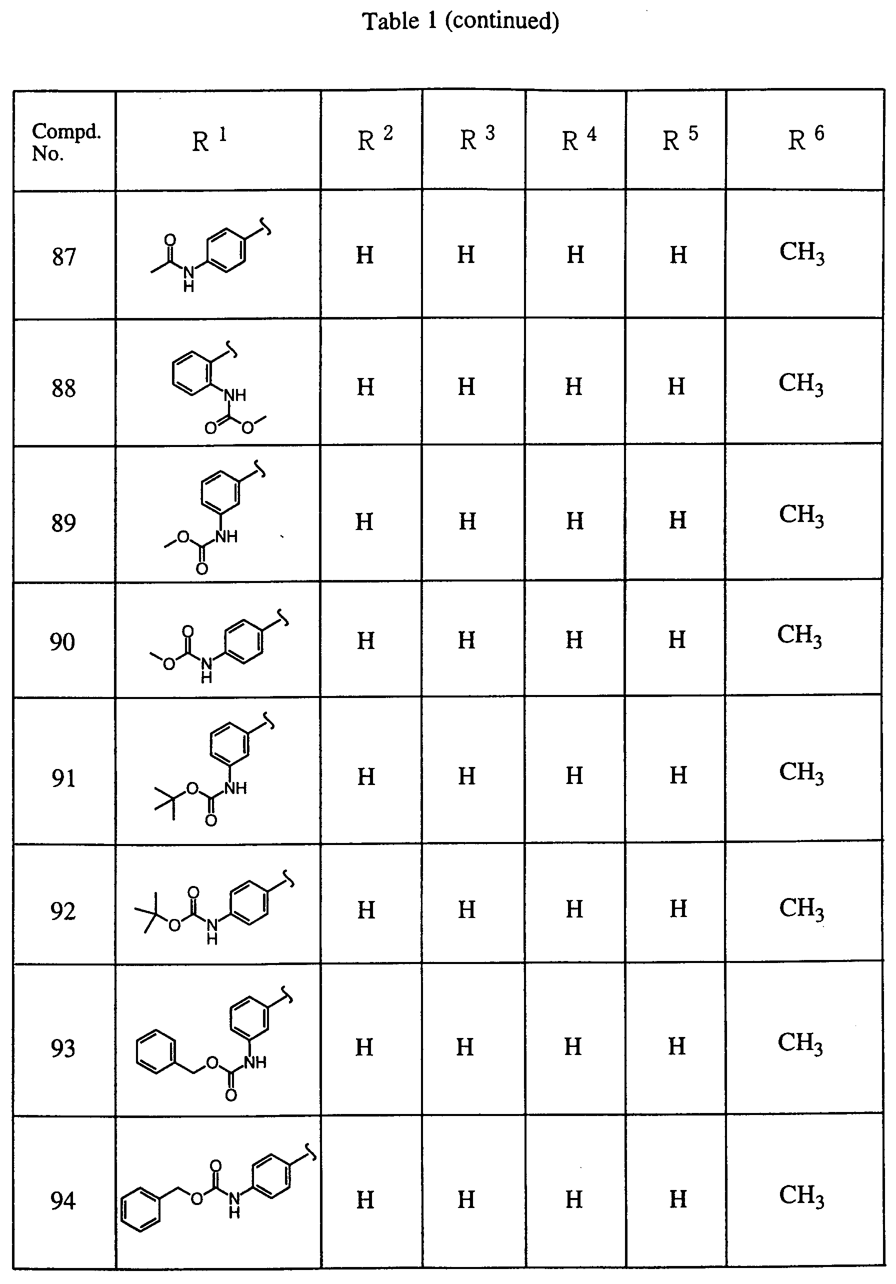

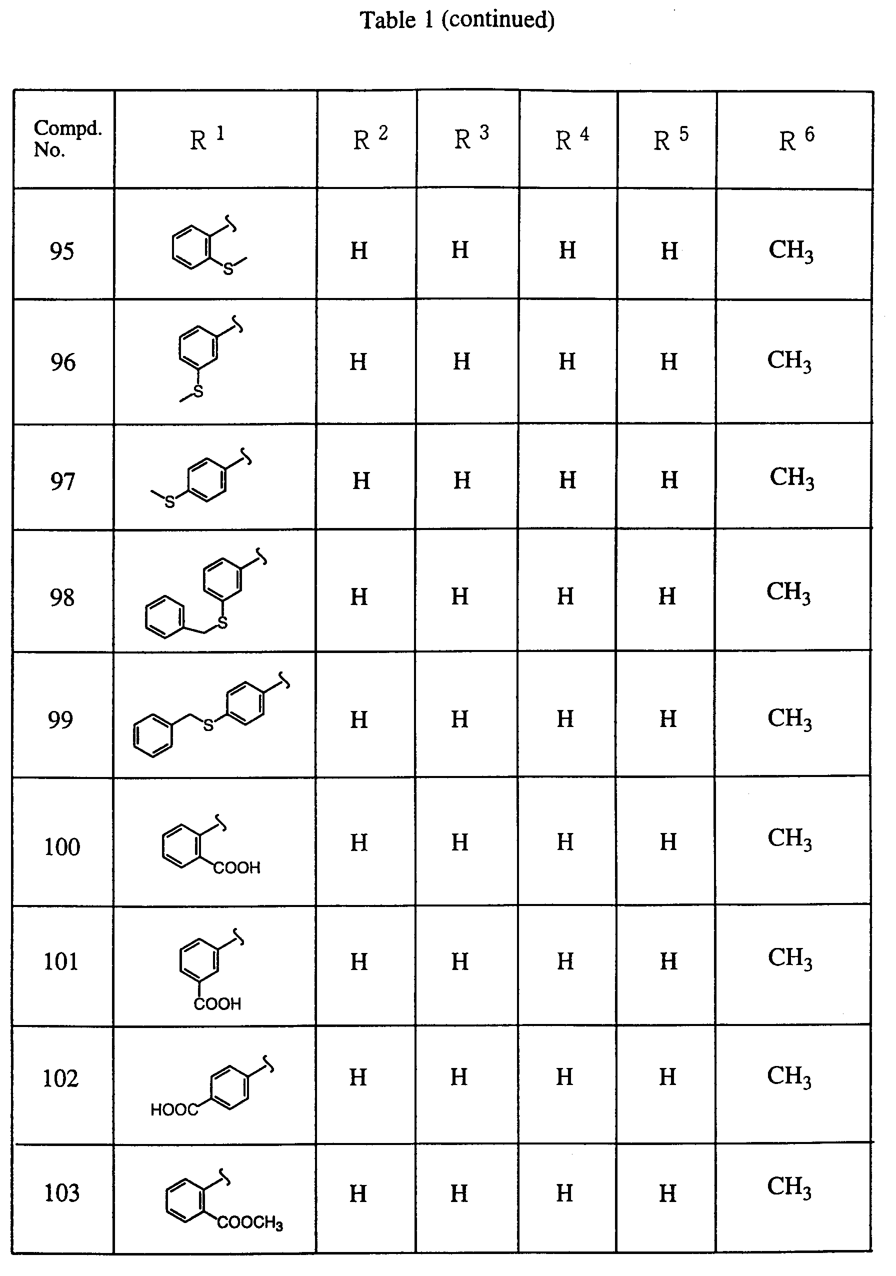

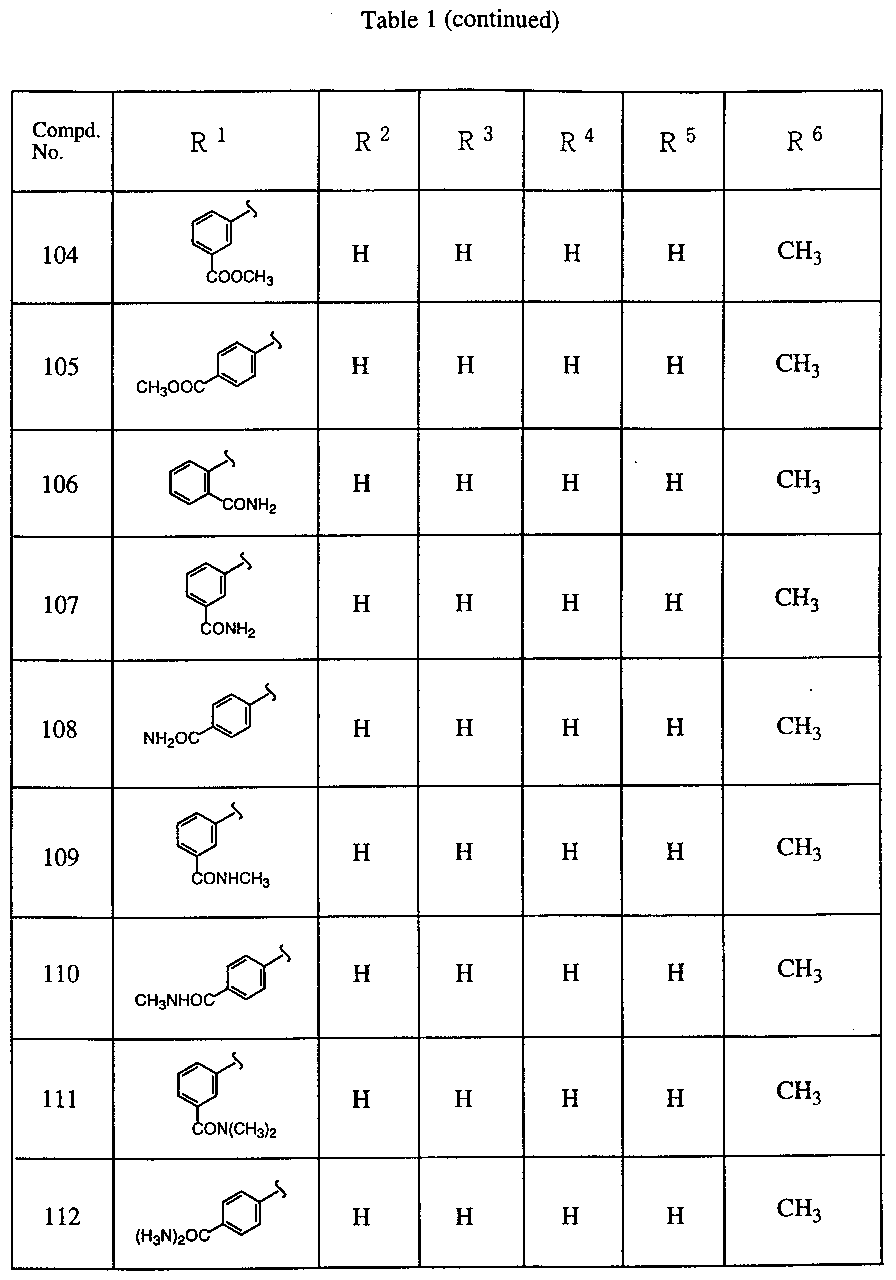

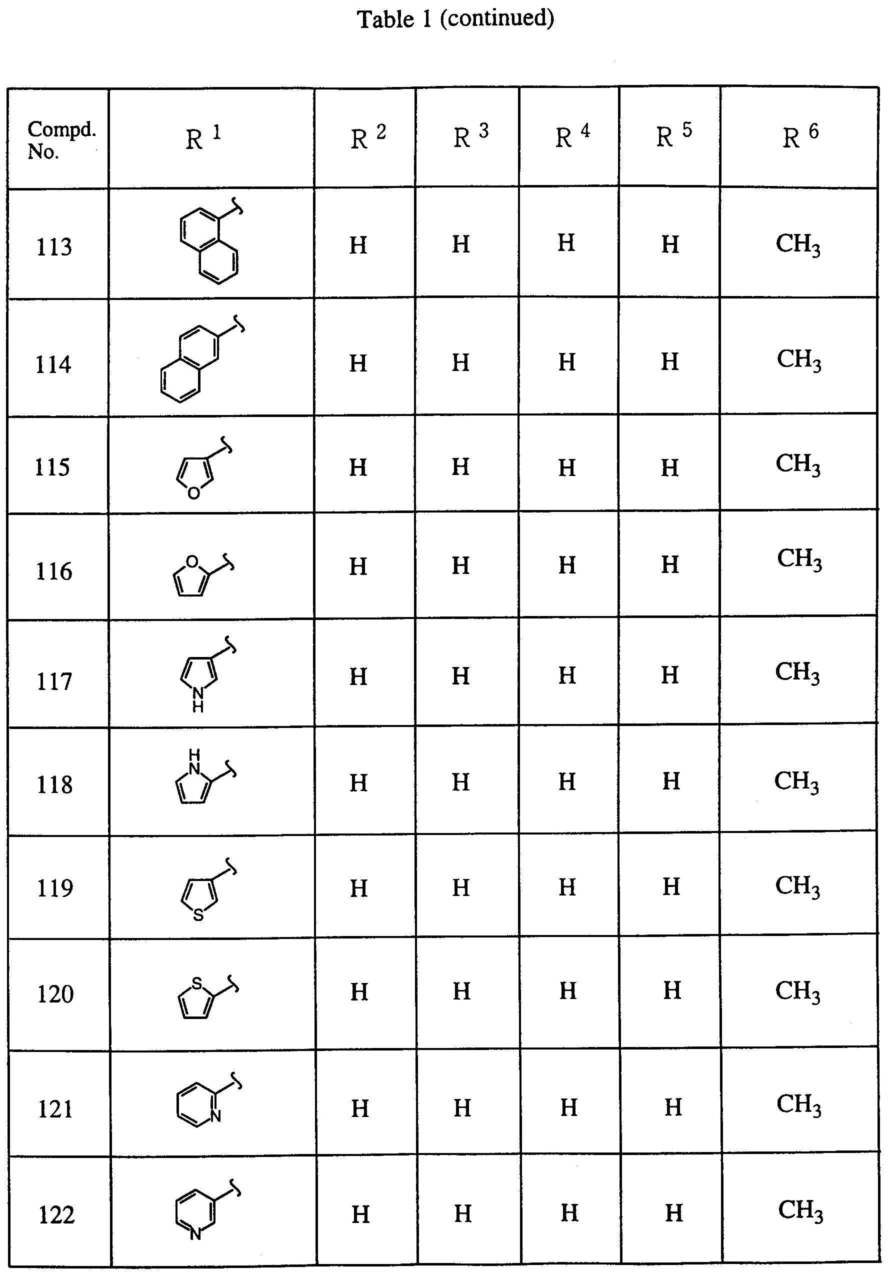

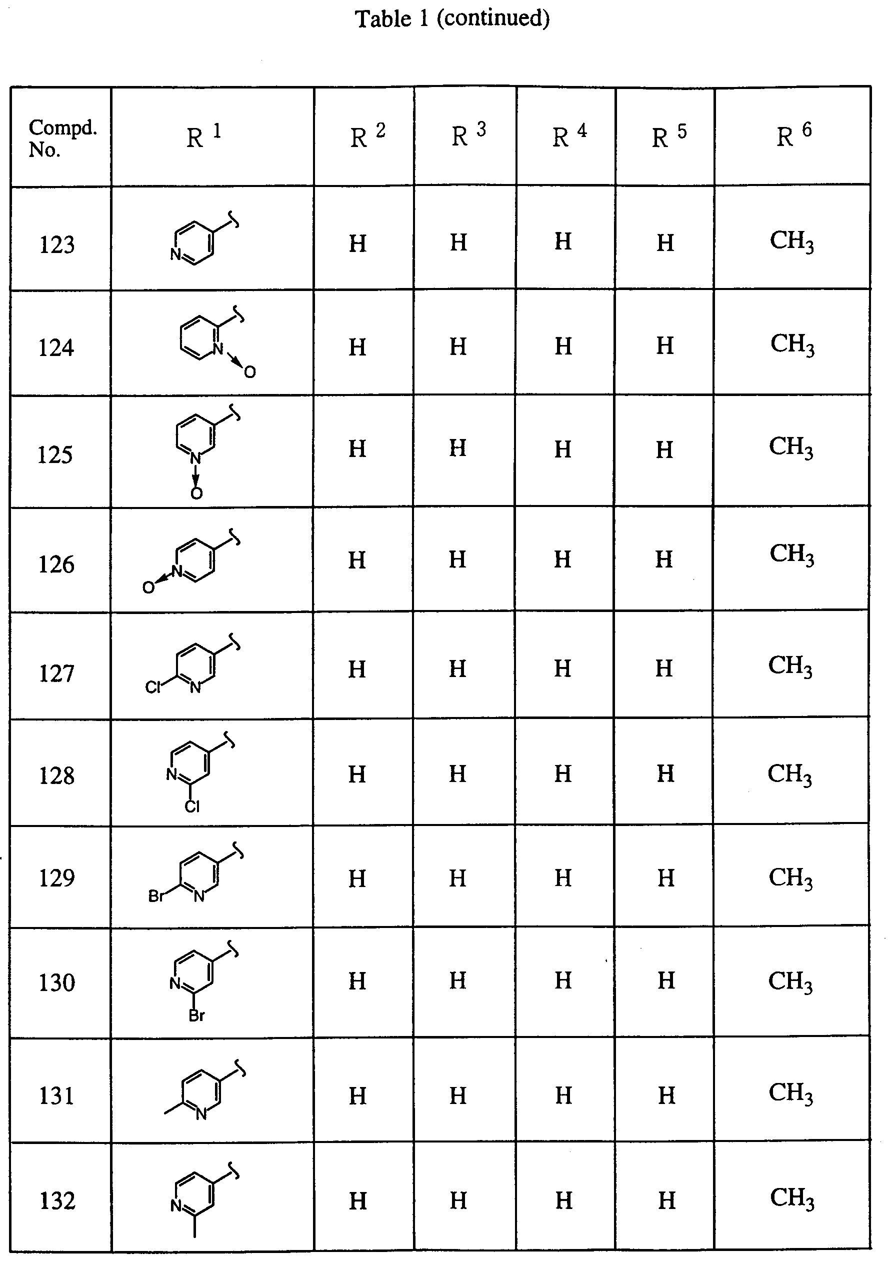

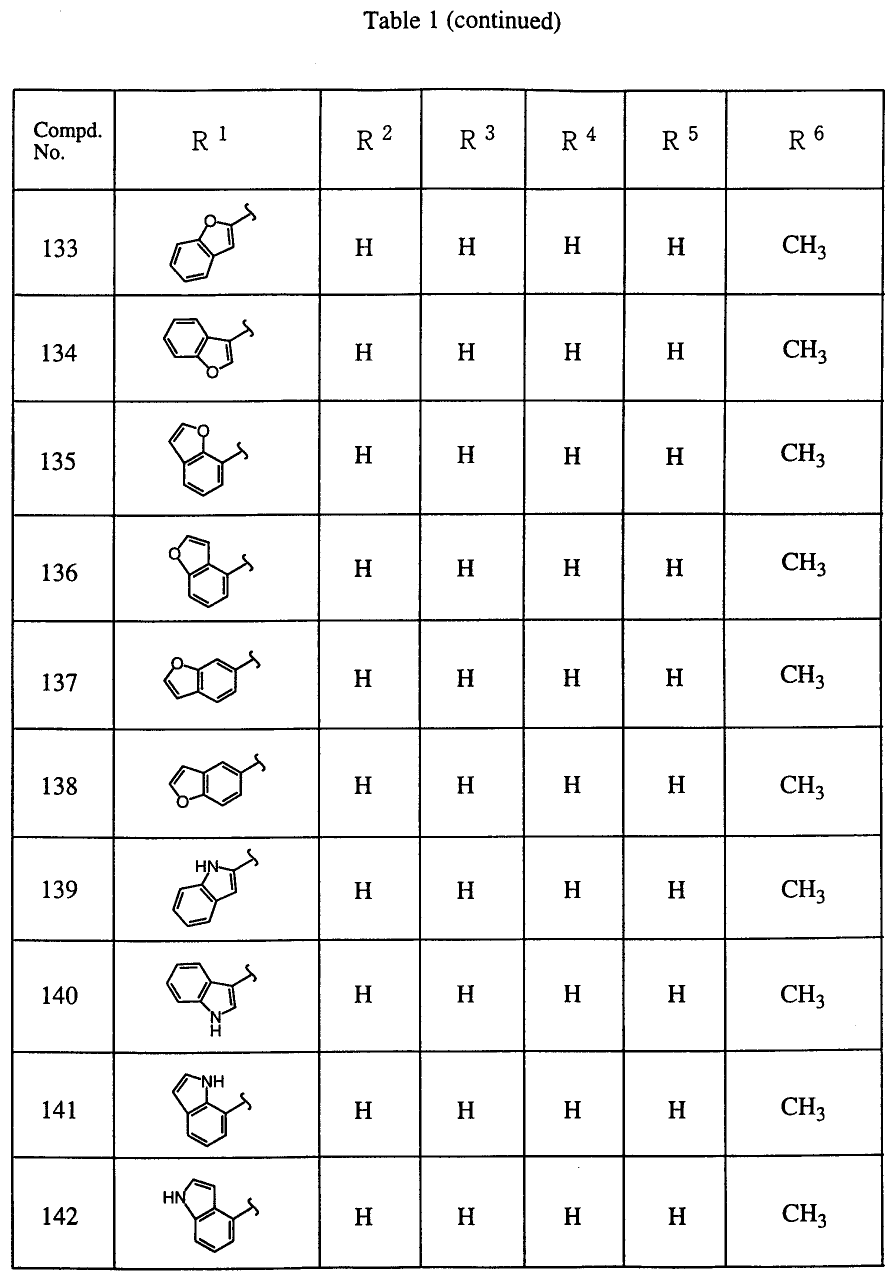

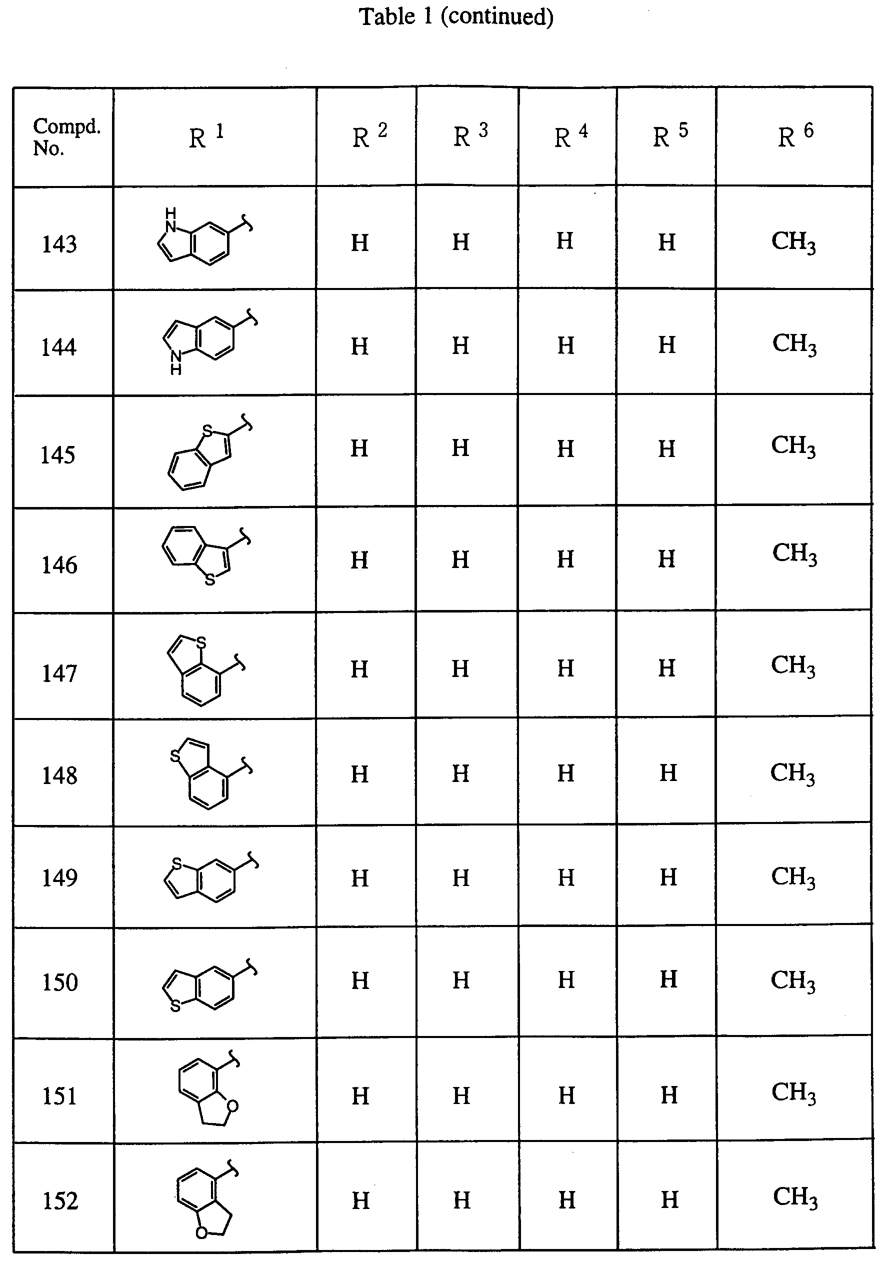

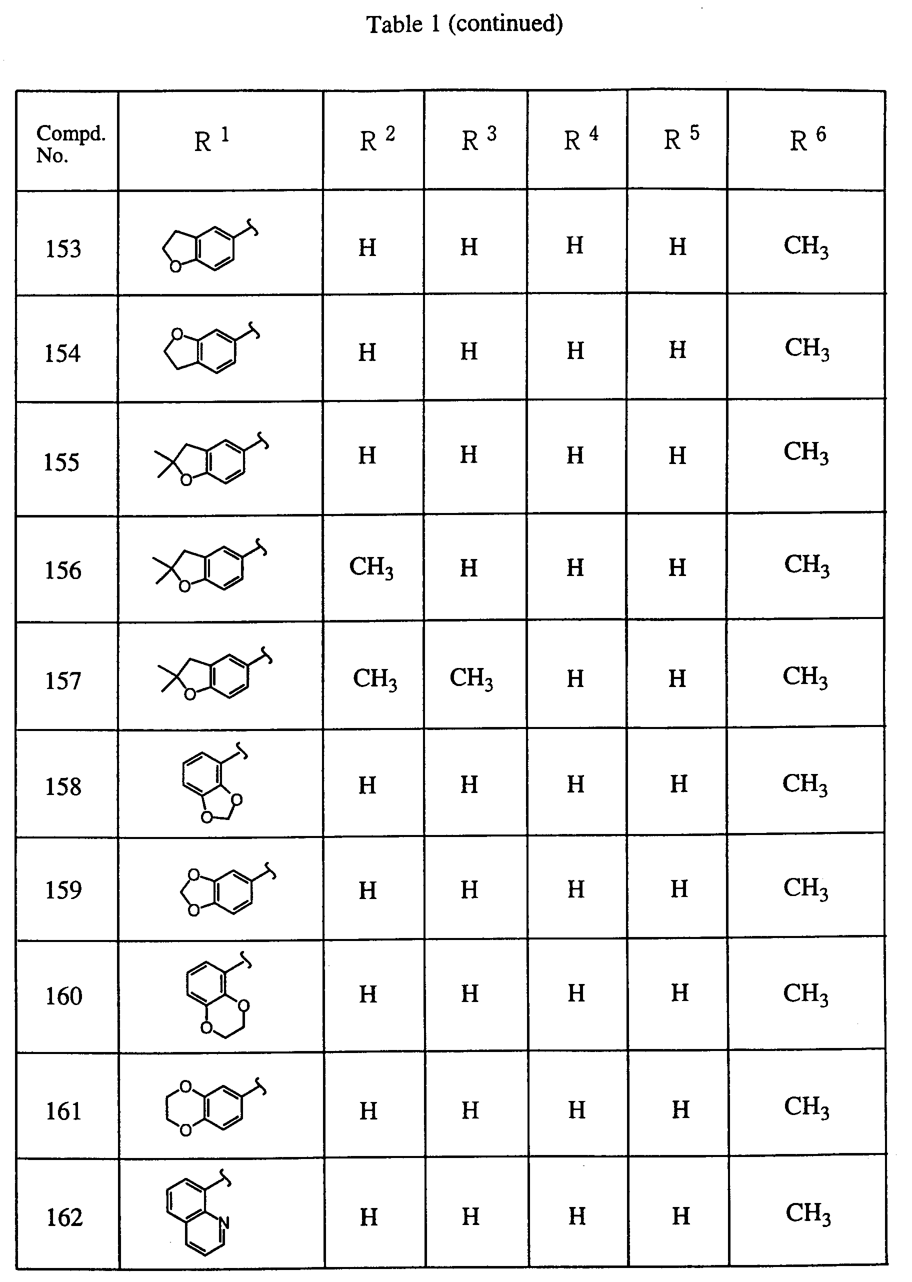

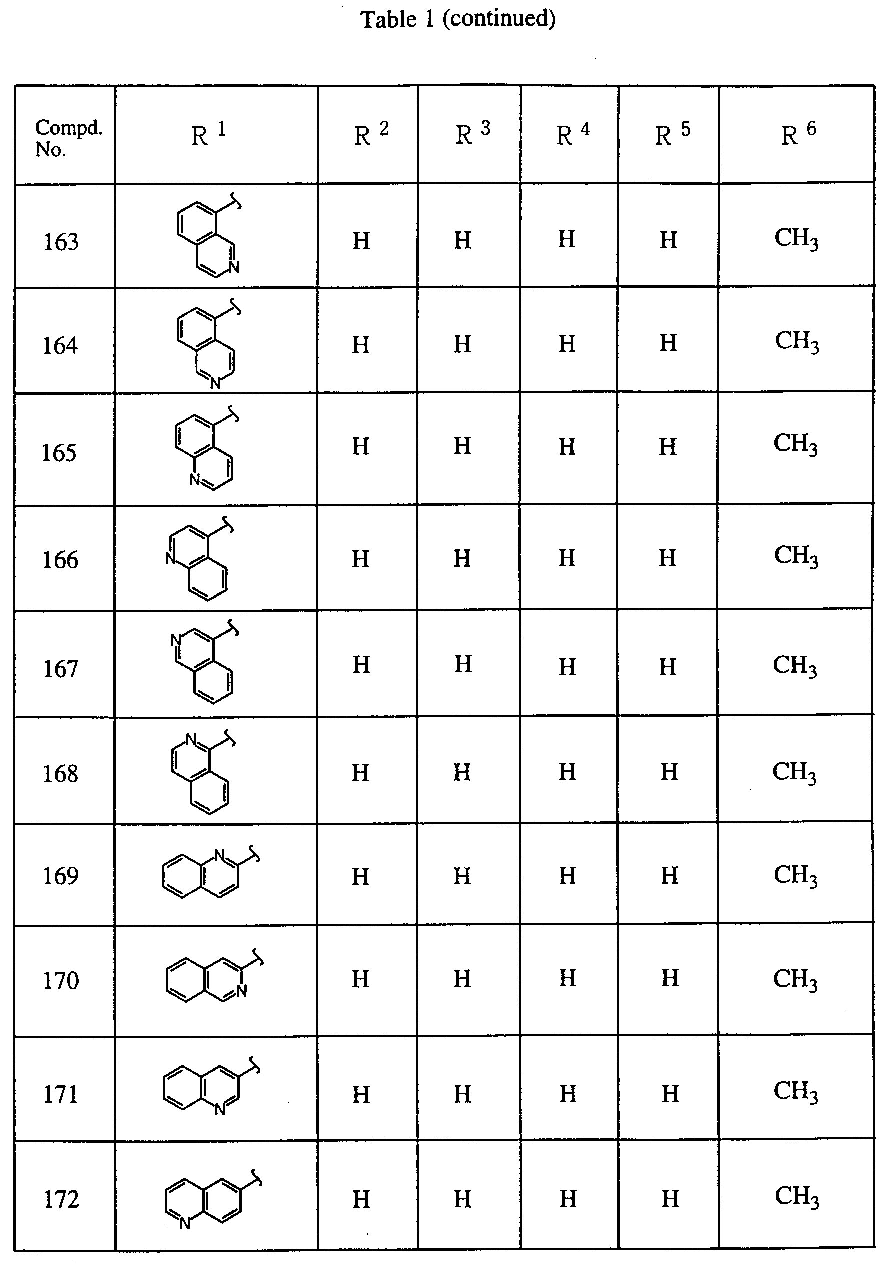

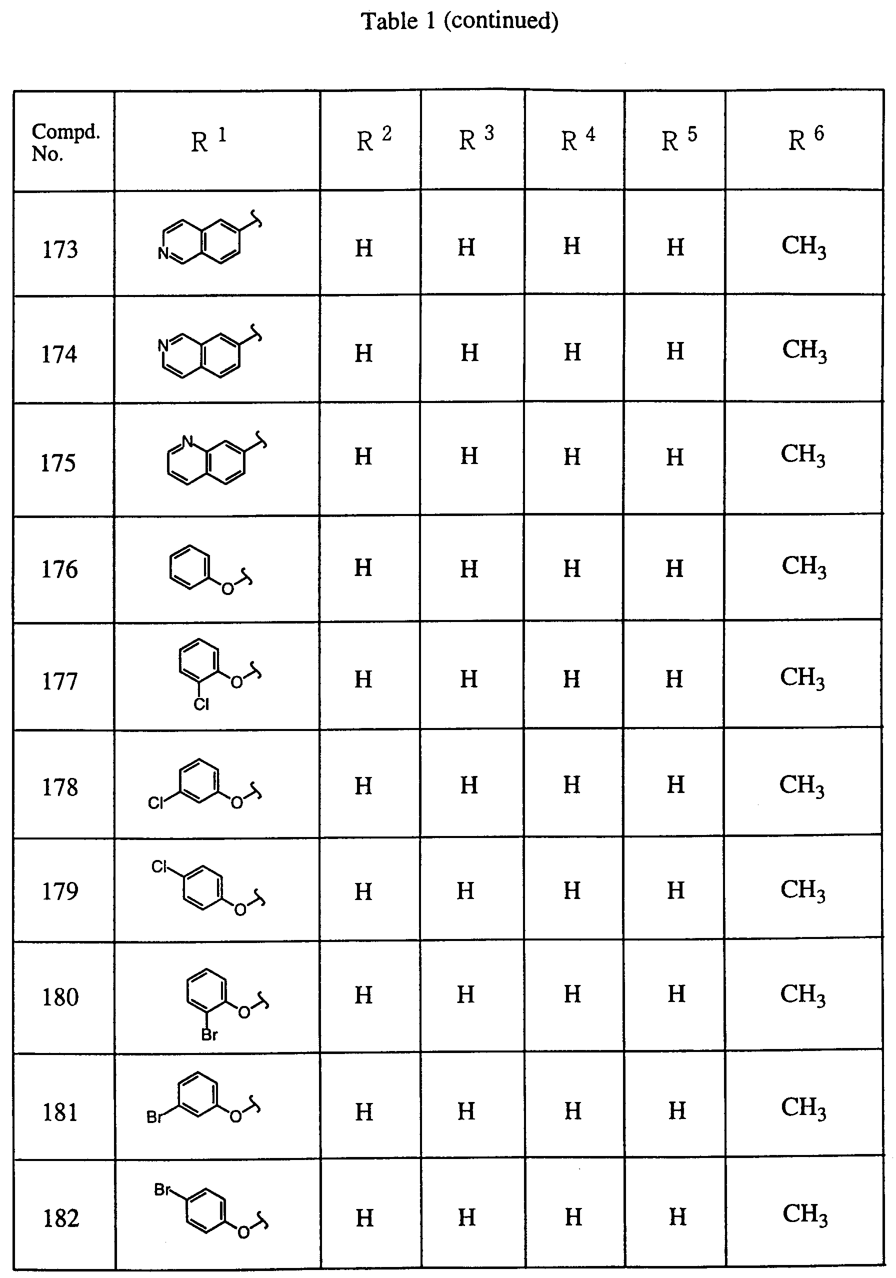

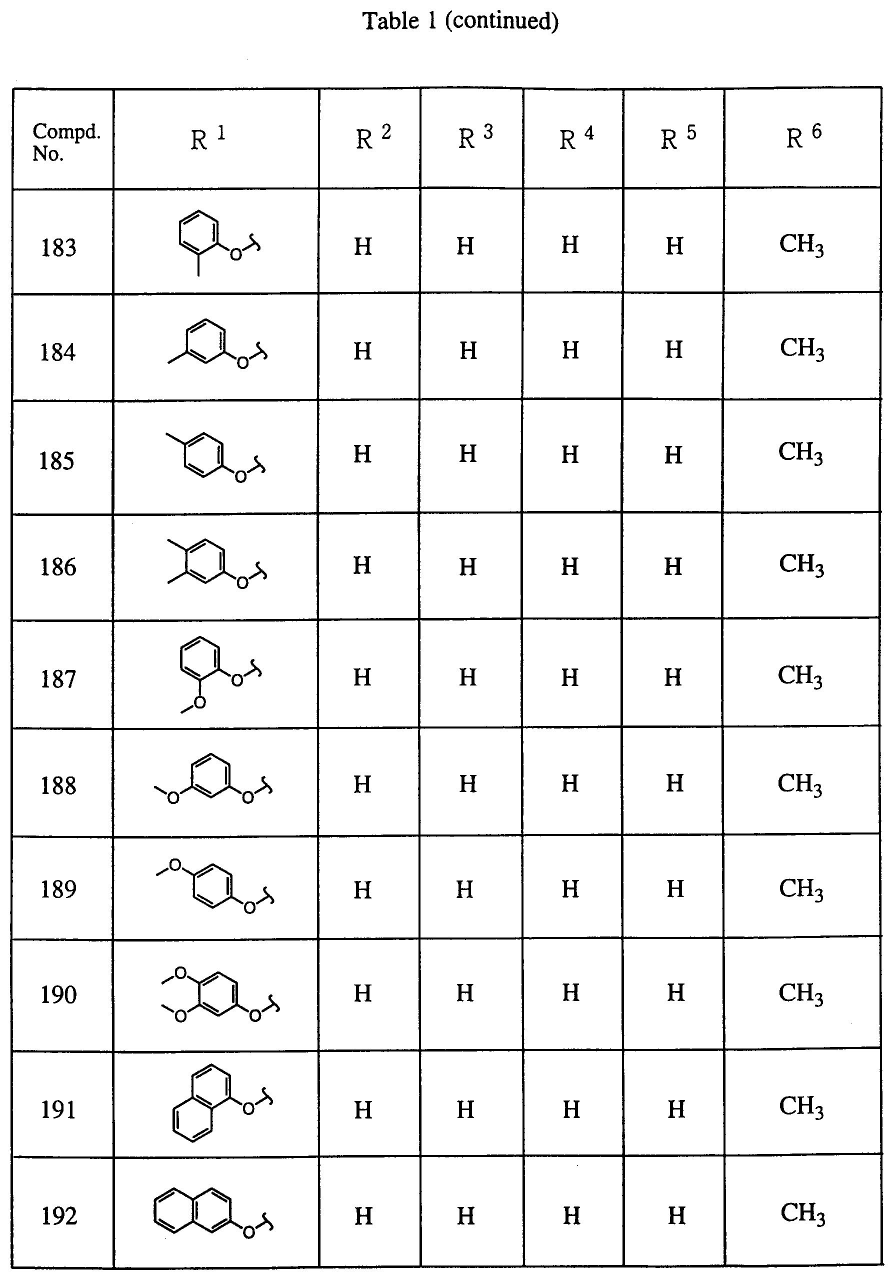

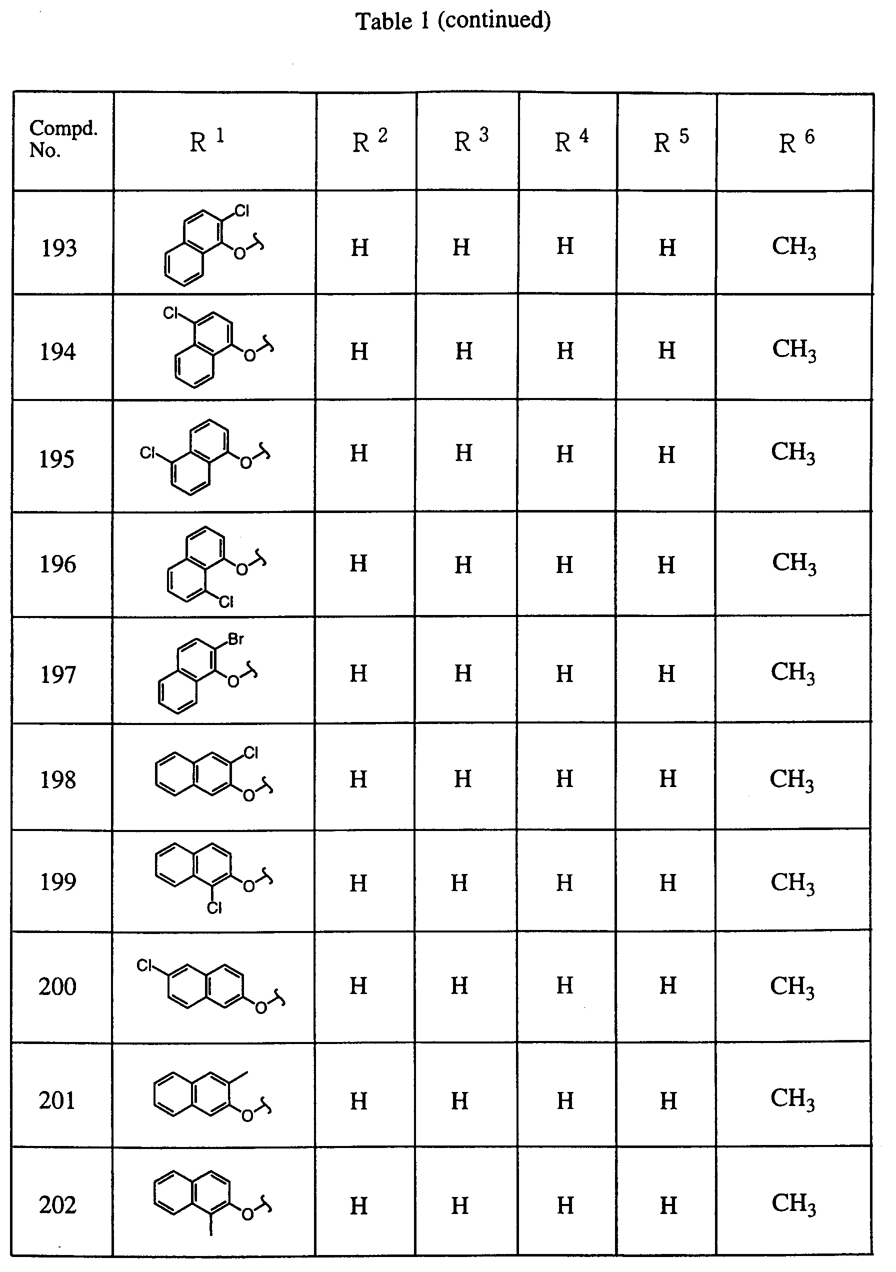

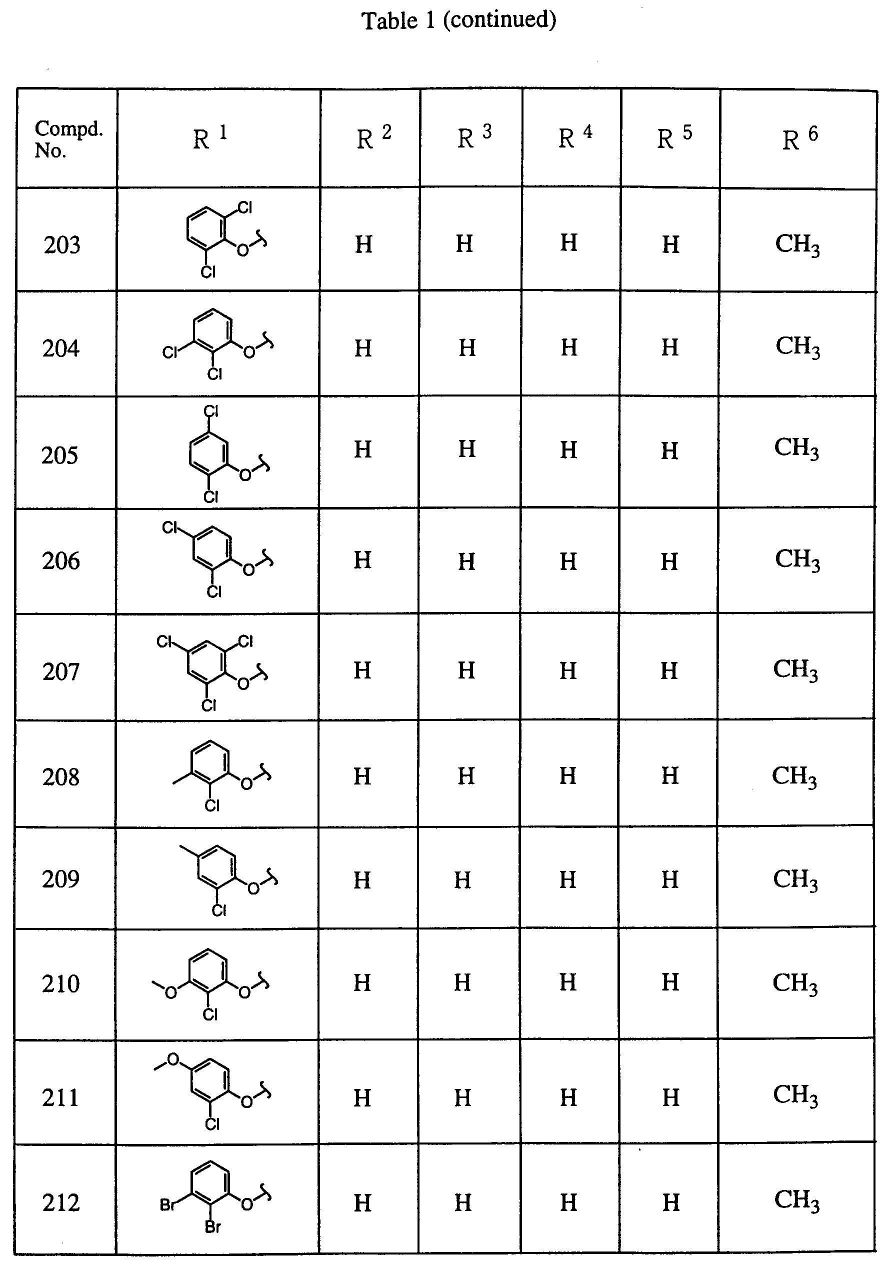

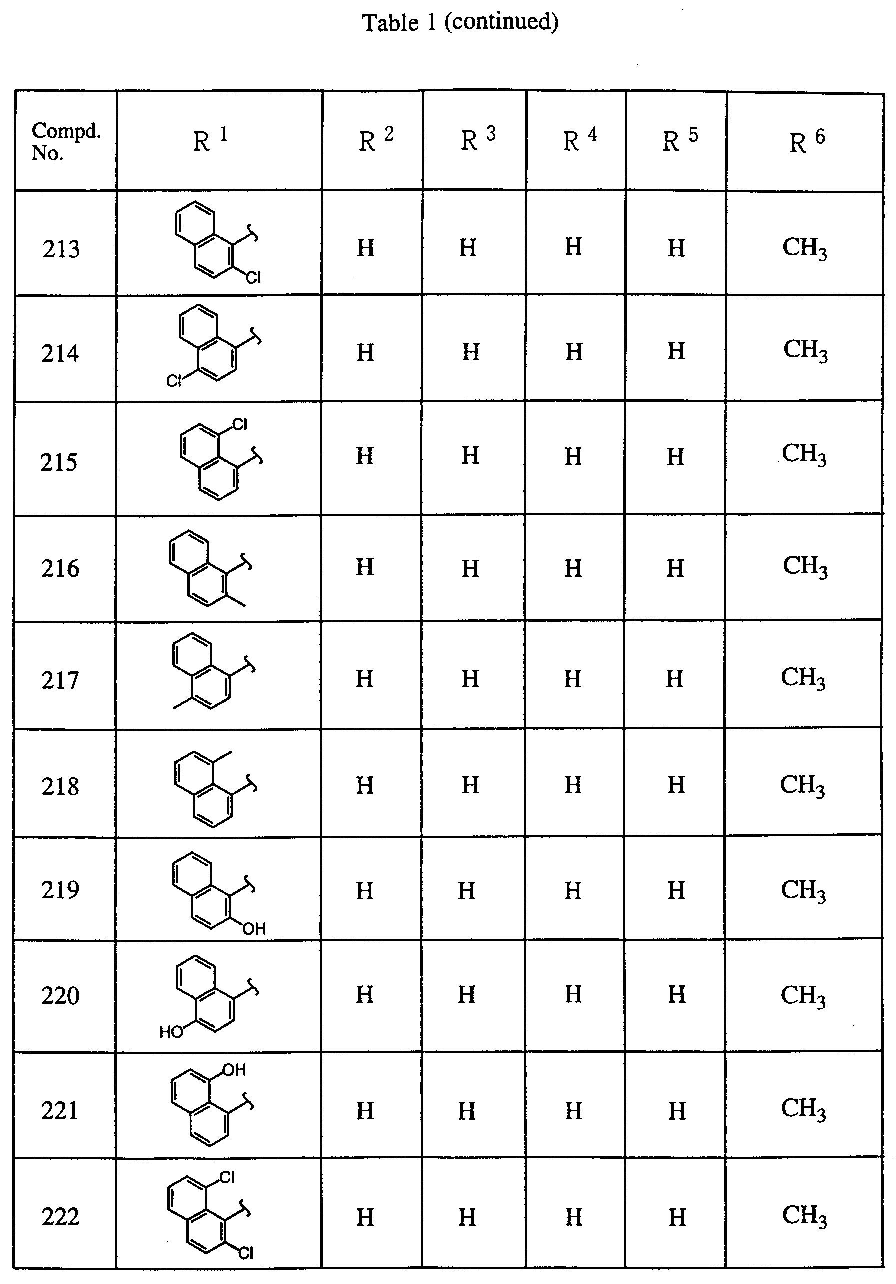

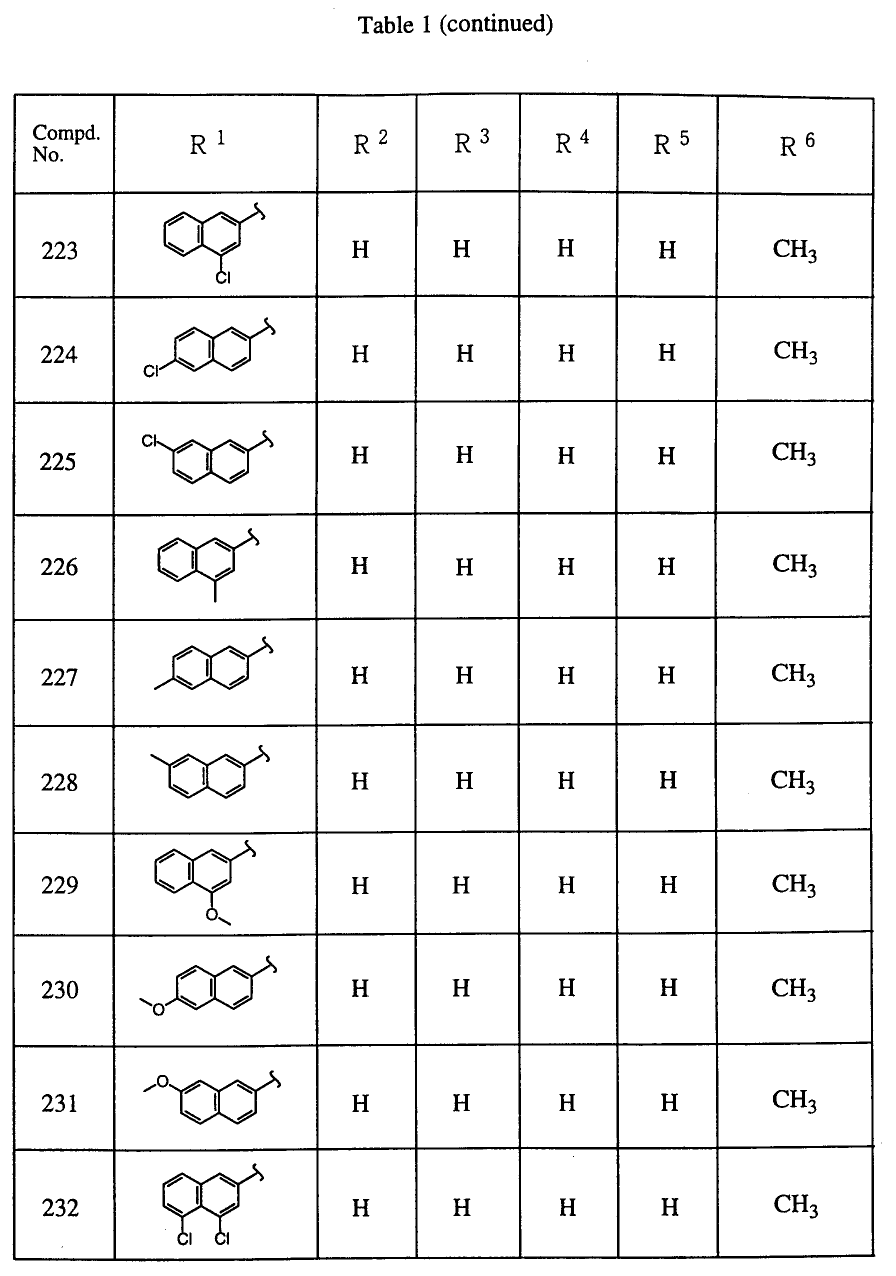

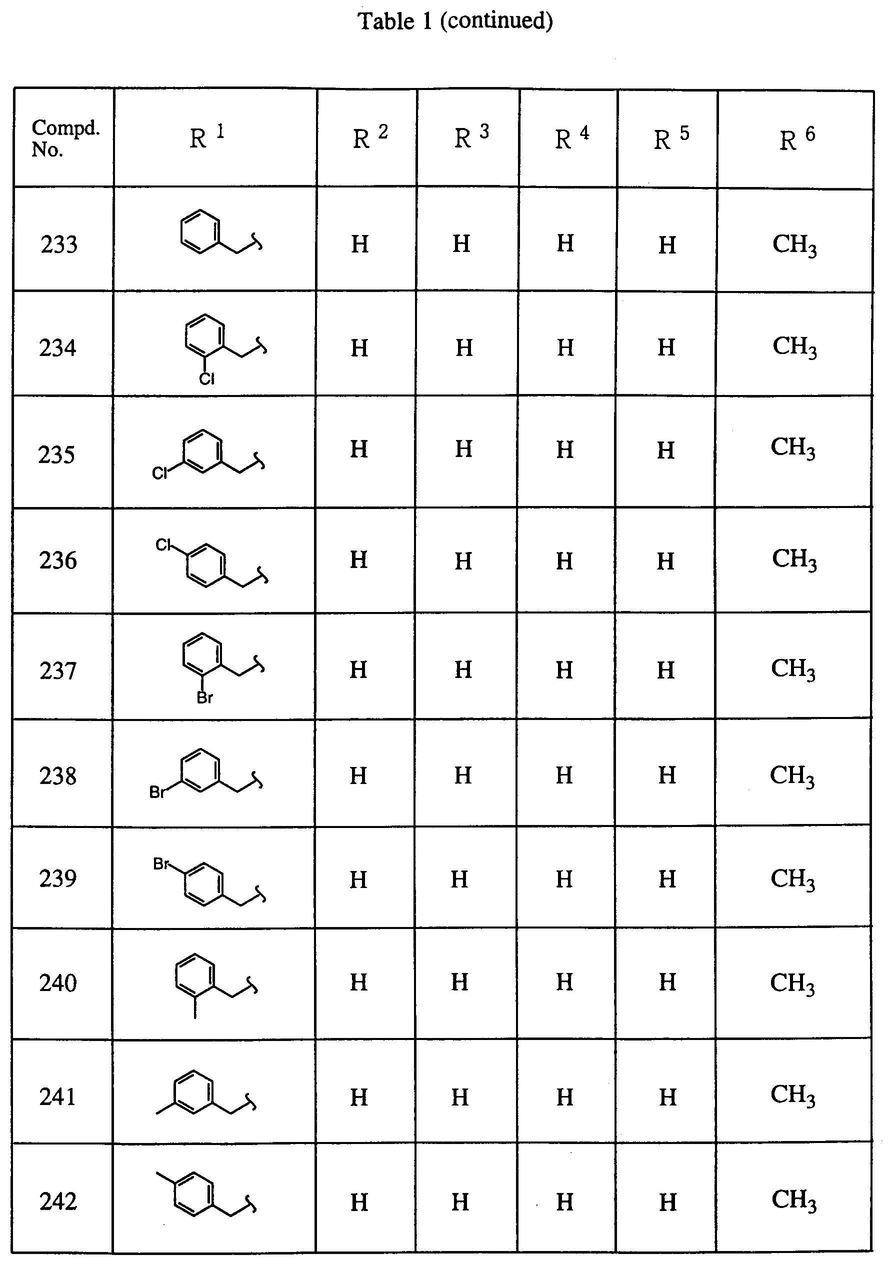

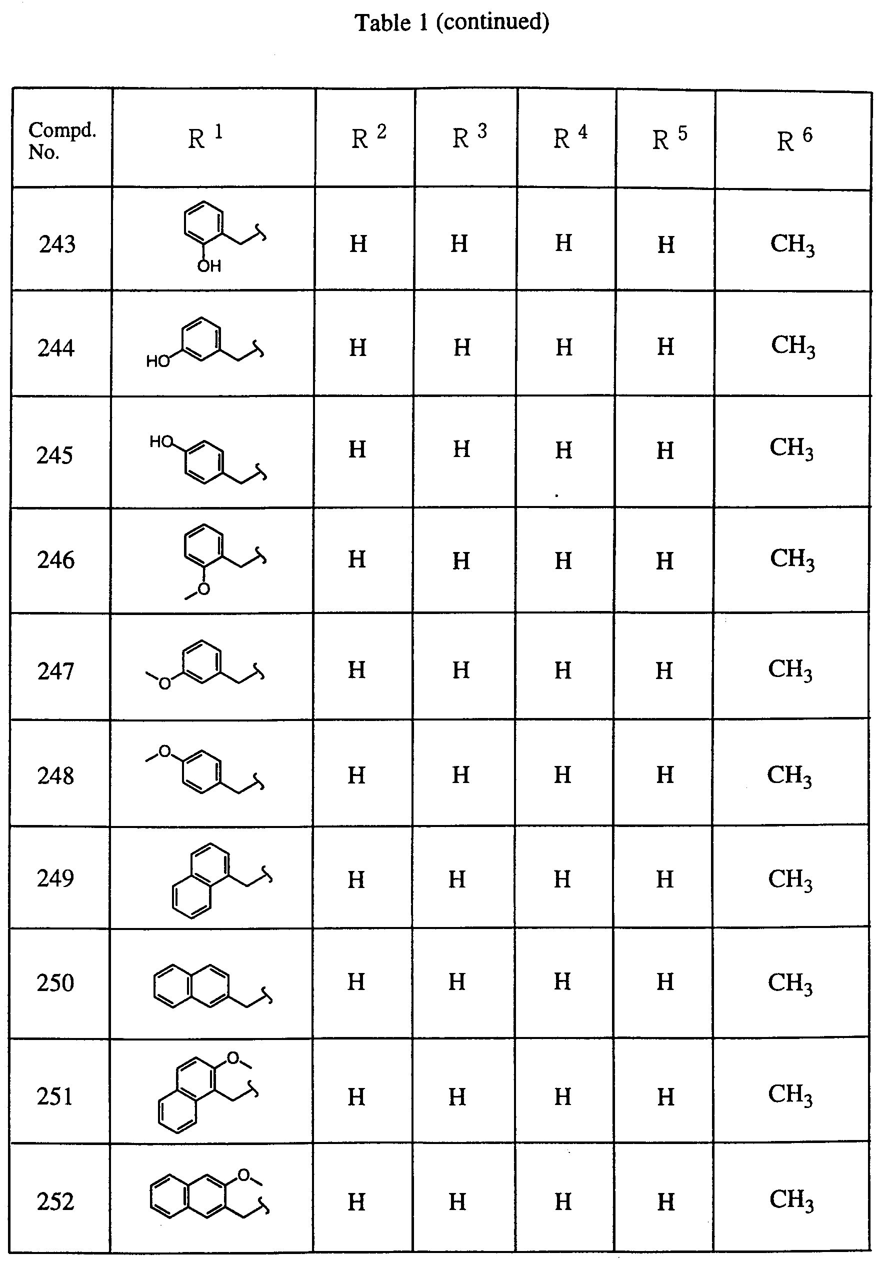

The amide derivatives of the present invention represented by the above general formula (I) can be prepared by, for example, the method explained below. 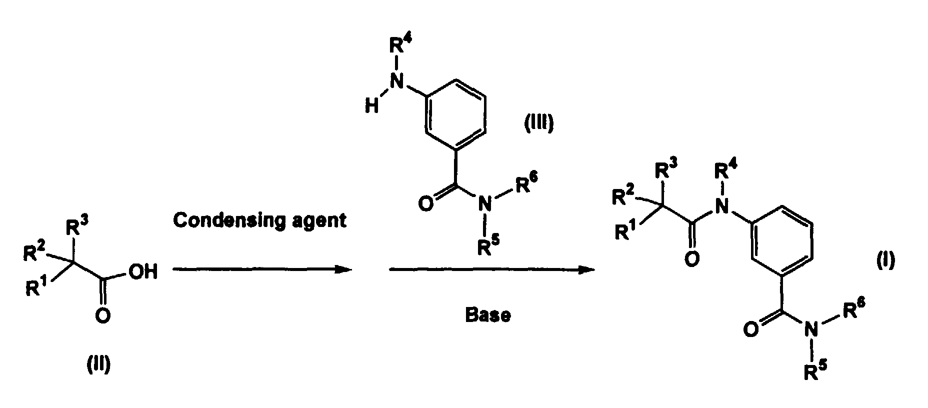 (In the above scheme, R¹, R², R³, R⁴, R⁵, and R⁶ are the same as those defined above.)

A carboxylic acid derivative represented by the above general formula (II) is allowed to react with a condensing agent such as dicyclohexylcarbodiimide, diphenylphosphoryl azide, carbonyldiimidazole, oxalyl chloride, isobutyl chloroformate, and thionyl chloride, optionally in the presence of a base such as triethylamine and pyridine as required, to activate a carboxylic acid, and then the resulting intermediate is allowed to react with an aniline derivative represented by the above general formula (III), optionally in the presence of a base such as triethylamine and pyridine as required, to obtain a compound represented by the above general formula (I). As a solvent used in the condensation reaction, a suitable solvent may be appropriately chosen depending on a type of a condensing agent. Reaction conditions may also be appropriately chosen so as to be suitable for a condensing agent used.

In the above series of reactions, protection and deprotection of one or more functional groups may sometimes be required. In such a case, a protective group suitable for each of the reactive functional group may be chosen, and reaction procedures can be employed according to known methods described in the literature.

The compounds of the present invention represented by the above general formula (I) have excellent antibacterial activity against Helicobacter pylori, and they can exhibit potent antibacterial activity against Helicobacter pylori in stomach. Accordingly, the medicaments of the present invention are useful for therapeutic and/or preventive treatment of various digestive diseases related to the infection caused by Helicobacter pylori, for example, a disease selected from the group consisting of gastritis, gastric ulcer, gastric cancer, gastric malignant lymphoma, MALT lymphoma, duodenal ulcer, and duodenal coma. More specifically, the compounds may preferably be used as medicaments for therapeutic treatment of gastritis, gastric ulcer and duodenal ulcer; medicaments for preventive treatment of gastric ulcer, duodenal ulcer, gastric malignant lymphoma, gastric cancer, and duodenal carcinoma; and medicaments for preventive treatment of recurrence of gastric ulcer and duodenal ulcer.

As an active ingredient of the medicament of the present invention, one or more substances selected from the group consisting of the compound represented by the above general formula (I) and a pharmaceutically acceptable salt thereof and a solvate thereof and a hydrate thereof can be used. The medicament of the present invention may preferably be provided in the form of a pharmaceutical composition comprising the above substance as an active ingredient and one or more pharmaceutically acceptable additives for pharmaceutical preparations. In the pharmaceutical compositions, a ratio of the active ingredient to the pharmaceutical additive may be about 1% by weight to about 90% by weight.

The medicament of the present invention may be administered as a pharmaceutical composition for oral administration such as granules, subtilized granules, powders, hard capsules, soft capsules, syrups, emulsions, suspensions, and liquid drugs, or administered as a pharmaceutical composition for parenteral administration such as sections for intravenous, intramuscular or subcutaneous administration, drip infusions, and suppositories. A preparation prepared as a powdery pharmaceutical composition may be dissolved before use and used as an injection or a drip infusion.

Solid or liquid pharmaceutical additives may be used for preparation of the pharmaceutical compositions. The pharmaceutical additives may be either organic or inorganic materials. Examples of excipients used for manufacturing solid preparations include, for example, lactose, sucrose, starch, talc, cellulose, dextrin, china clay, and calcium carbonate. For the manufacture of liquid formulations for oral administration such as emulsions, syrups, suspensions, and liquids, for example, ordinary inert diluents such as water and vegetable oils may be used. In addition to the inert diluents, auxiliaries such as, for example, moistening agents, spending aids, sweetening agents, aromatics, colorants, preservatives and the like may be formulated. Liquid preparations may be filled in capsules after their preparation that are made of an absorbable material such as gelatin. Examples of solvents or suspending mediums used for the manufacture of pharmaceutical preparations for parenteral administration such as injections and suppositories include, for example, water, propylene glycol, polyethylene glycol, benzyl alcohol, ethyl oleate, lecithin and the like. Examples of base materials used for preparation of suppositories include, for example, cacao butter, emulsified cacao butter, lauric lipid, Witepsol and the like. Methods for manufacturing the pharmaceutical preparations are not particularly limited, and any methods ordinarily used in the art may be employed.

A dose of the medicament of the present invention may generally be from 0.01 to 5,000 mg per day for an adult based on the weight of the compounds of the present invention. However, it is preferred to suitably increase or decreased depending on age, conditions, symptoms or other of a patient. The daily dose may be administered once a day or two to three times a day with suitable intervals, or alternatively, intermittently administered with intervals of several days. When used as an injection, a dose of the medicaments of the present invention may be about 0.001 to 100 mg per day for an adult based on the weight of the compounds of the present invention.

### Examples

The present invention will be explained more specifically by referring to the following examples. However, the scope of the present invention is not limited to these examples.

### Example 1: Preparation of N-(3-methylcarbamoylphenyl)-3-chlorophenylacetamide (Compound No. 17 in Table 1)

3-Chlorophenylacetic acid (192 mg) was dissolved in methylene chloride (8 ml), and oxalyl chloride (0.10 ml) and one drop of dimethylformamide were added to the solution. After stirring for 1 hour at room temperature, 3-aminobenzoylmethylamide (167 mg) and pyridine (0.19 ml) were added to the reaction mixture, and then stirred at room temperature overnight. After the solvent was evaporated under reduced pressure, water (10 ml) and 2N aqueous hydrochloric acid (1 ml) were added to the residue, and the deposited crystals were collected by filtration and washed with water. These crystals were dried and added in ethyl acetate (6 ml), and then the mixture was heated under reflux for ten minutes. The mixture was cooled to room temperature, and the crystals were collected by filtration and washed with ethyl acetate to obtain the desired compound (233 mg, yield 68%).
Melting point: 165-166°C
IR (KBr, cm⁻¹): 3324, 1642, 1593, 1555.
NMR (DMSO-d₆, δ): 2.76 (d, J=4.5Hz, 3H), 3.68 (s, 2H), 7.25-7.42 (m, 5H), 7.48 (d, J=7.8 Hz, 1H), 7.74 (d, J=7.8Hz, 1H), 8.02 (dd, J=1.8Hz, 1.8Hz, 1H), 8.36 (d, J=4.5Hz, 1H), 10.31 (s, 1H).

In similar manners to the method of Example 1, compounds of Examples 2-50 were prepared. Their physicochemical properties are set out below.

### Example 2: Preparation of N-(3-methylcarbamoylphenyl)cyclohexylacetamide (Compound No. 4 in Table 1)

Melting point: 183°C
IR (KBr, cm⁻¹): 3293, 1657, 1640, 1588, 1535.
NMR (DMSO-d₆, δ): 0.99 (m, 2H), 1.03-1.38 (m, 3H), 1.55-1.90 (m, 6H), 2.19 (d, J=7.0Hz, 2H), 2.76 (d, J=4.5Hz, 3H), 7.34 (dd, J=7.8Hz, 7.8Hz, 1H), 7.45 (d, J=7.8Hz, 1H), 7.74 (d, J=7.8Hz, 1H), 8.01 (s, 1H), 8.34 (d, J=4.5Hz, 1H), 9.95 (s, 1H).

### Example 3: Preparation of N-(3-methylcarbamoylphenyl)phenylacetamide (Compound No. 7 in Table 1)

Melting point: 140-142°C
NMR (DMSO-d₆, δ): 2.75 (d, J=4.5Hz, 3H), 3.63 (s, 2H), 7.22-7.48 (m, 7H), 7.74 (m, 1H), 8.01 (s, 1H), 8.37 (d, J=4.5Hz, 1H), 10.30 (s, 1H).

### Example 4: Preparation of N-(3-methylcarbamoylphenyl)-1-phenylcyclopentanecarboxamide (Compound No. 11 in Table 1)

Melting point: 147°C
IR (KBr, cm⁻¹): 3339, 3275, 1638, 1586, 1557, 1528.
NMR (DMSO-d₆, δ): 1.67 (m, 4H), 1.94 (m, 2H), 2.65 (m, 2H), 2.75 (d, J=4.5Hz, 3H), 7.20-7.60 (m, 7H), 7.76 (d, J=7.2Hz, 1H), 7.98 (s, 1H), 8.33 (d, J=4.5Hz, 1H), 9.32 (s, 1H).

### Example 5: Preparation of N-(3-methylcarbamoylphenyl)-3-fluorophenylacetamide (Compound No. 14 in Table 1)

Melting point: 147-148°C
IR (KBr, cm⁻¹): 3314, 1661, 1636, 1587, 1530.
NMR (DMSO-d₆, δ): 2.76 (d, J=4.2Hz, 3H), 3.69 (s, 2H), 7.08 (dd, J=5.7Hz, 5.7Hz, 1H), 7.14 (d, J=7.5Hz, 2H), 7.38 (m, 2H), 7.47 (d, J=8.1Hz, 1H), 7.74 (d, J=8.1Hz, 1H), 8.02 (s, 1H), 8.35 (d, J=4.2Hz, 1H), 10.30 (s, 1H).

### Example 6: Preparation of N-(3-methylcarbamoylphenyl)-4-fluorophenylacetamide (Compound No. 15 in Table 1)

Melting point: 155-156°C
IR (KBr, cm⁻¹): 3293, 1657, 1634, 1588, 1535, 1512.
NMR (DMSO-d₆, δ): 2.76 (d, J=3.9Hz, 3H), 3.65 (s, 2H), 7.15 (dd, J=9.0Hz, 9.0Hz, 2H), 7.25-7.41 (m, 3H), 7.47 (d, J=7.5Hz, 1H), 7.75 (d, J=8.1Hz, 1H), 8.02 (s, 1H), 8.35 (d, J=3.9Hz, 1H), 10.28 (s, 1H).

### Example 7: Preparation of N-(3-methylcarbamoylphenyl)-2-chlorophenylacetamide (Compound No. 16 in Table 1)

Melting point: 211-212°C
IR (KBr, cm⁻¹): 3268, 1659, 1642, 1586, 1535.
NMR (DMSO-d₆, δ): 2.77 (d, J=3.6Hz, 3H), 3.85 (s, 2H), 7.25-7.55 (m, 6H), 7.74 (d, J=7.5Hz, 1H), 8.04 (s, 1H), 8.36 (d, J=3.6Hz, 1H), 10.34 (s, 1H).

### Example 8: Preparation of N-(3-methylcarbamoylphenyl)-4-chlorophenylacetamide (Compound No. 18 in Table 1)

Melting point: 163-164°C
IR (KBr, cm⁻¹): 3279, 1663, 1640, 1588, 1535.
NMR (DMSO-d₆, δ): 2.76 (d, J=3.9Hz, 3H), 3.66 (s, 2H), 7.35-7.42 (m, 5H), 7.47 (d, J=7.8Hz, 1H), 7.75 (d, J=7.8Hz, 1H), 8.02 (dd, J=1.5Hz, 1.5Hz, 1H), 8.36 (d, J=3.9Hz, 1H), 10.30 (s, 1H).

### Example 9: Preparation of N-(3-carbamoylphenyl)-3-bromophenylacetamide (Compound No. 20 in Table 1)

Melting point: 202°C
IR (KBr, cm⁻¹): 3378, 3295, 1659, 1624, 1586, 1534.
NMR (DMSO-d₆, δ): 3.67 (s, 2H), 7.20-7.60 (m, 7H), 7.76 (d, J=9.3Hz, 1H), 7.94 (s, 1H), 8.03 (s, 1H), 10.33 (s, 1H).

### Example 10: Preparation of N-(3-methylcarbamoylphenyl)-3-bromophenylacetamide (Compound No. 22 in Table 1)

Melting point: 176-178°C
IR (KBr, cm⁻¹): 3324, 3254, 1642, 1591, 1554.
NMR (DMSO-d₆, δ): 2.76 (d, J=4.5Hz, 3H), 3.68 (s, 2H), 77.27-7.41 (m, 3H), 7.45-7.50 (m, 2H), 7.56 (s, 1H), 7.75 (d, J=8.0Hz, 1H), 8.03 (s, 1H), 8.42 (d, J=4.5Hz, 1H), 10.35 (s, 1H).

### Example 11: Preparation of N-(3-dimethylcarbamoylphenyl)-3-bromophenylacetamide (Compound No. 23 in Table 1)

Melting point: 119-120°C
IR (KBr, cm⁻¹): 1678, 1613, 1588, 1557.
NMR (DMSO-d₆, δ): 2.90 (s, 3H), 2.96 (s, 3H), 3.68 (s, 2H), 7.06 (d, J=7.8Hz, 1H), 7.25-7.41 (m, 3H), 7.47 (m, 1H), 7.53-7.60 (m, 2H), 7.68 (s, 1H), 10.30 (s, 1H).

### Example 12: Preparation of N-(3-ethylcarbamoylphenyl)-3-bromophenylacetamide (Compound No. 24 in Table 1)

Melting point: 155°C
IR (KBr, cm⁻¹): 3329, 3268, 1665, 1640, 1549.
NMR (DMSO-d₆, δ): 1.11 (t, J=6.9Hz, 3H), 3.29 (m, 2H), 3.67 (s, 2H), 7.20-7.40 (m, 3H), 7.47 (d, J=8.1Hz, 1H), 7.49 (d, J=8.1Hz, 1H), 7.56 (s, 1H), 7.75 (d, J=8.4Hz, 1H), 8.00 (s, 1H), 8.41 (t, J=5.1Hz, 1H), 10.32 (s, 1H).

### Example 13: Preparation of N-(3-(2-hydroxyethyl)carbamoylphenyl)-3-bromophenylacetamide (Compound No. 28 in Table 1)

Melting point: 202°C
IR (KBr, cm⁻¹): 3407, 3358, 3279, 1671, 1640, 1589, 1539.
NMR (DMSO-d₆, δ): 3.26 (m, 2H), 3.47 (m, 2H), 3.65 (s, 2H), 4.67 (t, J=5.7Hz, 1H), 7.20-7.60 (m, 6H), 7.73 (d, J=7.2Hz, 1H), 7.99 (s, 1H), 8.32 (m, 1H), 10.28 (s, 1H).

### Example 14: Preparation of N-(3-hydroxycarbamoylphenyl)-3-bromophenylacetamide (Compound No. 29 in Table 1)

Melting point: 184-186°C (decomposition)
IR (KBr, cm⁻¹): 3314, 3231, 1663, 1632, 1582, 1535.
NMR (DMSO-d₆, δ): 3.68 (s, 2H), 7.25-7.60 (m, 6H), 7.75 (d, J=6.9Hz, 1H), 7.98 (s, 1H), 9.01 (s, 1H), 10.33 (s, 1H), 11.12 (s, 1H).

### Example 15: Preparation of N-(methoxycarbamoylphenyl)-3-bromophenylacetamide (Compound No. 30 in Table 1)

Melting point: 166°C
IR (KBr, cm⁻¹): 3299, 3187, 1659, 1611, 1595, 1560.
NMR (DMSO-d₆, δ): 3.69 (s, 5H), 7.22-7.60 (m, 6H), 7.77 (s, 1H), 8.00 (s, 1H), 10.37 (s, 1H), 11.69 (s, 1H).

### Example 16: Preparation of N-(3-methylcarbamoylphenyl)-4-bromophenylacetamide (Compound No. 33 in Table 1)

Melting point: 165-166°C
IR (KBr, cm⁻¹): 3283, 1665, 1642, 1588, 1534.
NMR (DMSO-d₆, δ): 2.77 (d, J=4.5Hz, 3H), 3.64 (s, 2H), 7.23-7.40 (m, 3H), 7.40-7.58 (m, 3H), 7.75 (d, J=7.8Hz, 1H), 8.01 (s, 1H), 8.37 (d, J=4.5Hz, 1H), 10.30 (s, 1H).

### Example 17: Preparation of N-(3-methylcarbamoylphenyl)-3-methylphenylacetamide (Compound No. 42 in Table 1)

Melting point: 131°C
IR (KBr, cm⁻¹): 3299, 1659, 1634, 1586, 1530.
NMR (DMSO-d₆, δ): 2.29 (s, 3H), 2.76 (d, J=4.5Hz, 3H), 3.60 (s, 2H), 7.06 (d, J=6.9Hz, 1H), 7.09-7.22 (m, 3H), 7.36 (dd, J=7.8Hz, 7.8Hz, 1H), 7.47 (d, J=7.8Hz, 1H), 7.75 (d, J=7.8Hz, 1H), 8.02 (s, 1H), 8.35 (d, J=4.5Hz, 1H), 10.26 (s, 1H).

### Example 18: Preparation of N-(3-methylcarbamoylphenyl)-4-methylphenylacetamide (Compound No. 43 in Table 1)

Melting point: 174-175°C
IR (KBr, cm⁻¹): 3339, 3295, 1659, 1639, 1586, 1528.
NMR (DMSO-d₆, δ): 2.27 (s, 3H), 2.76 (d, J=4.5Hz, 3H), 3.59 (s, 2H), 7.12 (d, J=8.1Hz, 2H), 7.22 (d, J=8.1Hz, 2H), 7.36 (dd, J=7.8Hz, 7.8Hz, 1H), 7.47 (d, J=7.8Hz, 1H), 7.75 (d, J=7.8Hz, 1H), 8.01 (s, 1H), 8.35 (d, J=4.5Hz, 1H), 10.24 (s, 1H).

### Example 19: Preparation of N-(3-methylcarbamoylphenyl)-3-methoxyphenylacetamide (Compound No. 59 in Table 1)

Melting point: 104-106°C
NMR (DMSO-d₆, δ): 2.76 (d, J=4.5Hz, 3H), 3.60 (s, 2H), 3.73 (s, 3H), 6.81 (m, 1H), 6.89-6.92 (m, 2H), 7.23 (m, 2H), 7.35 (m, 1H), 7.47 (m, 1H), 7.76 (m, 1H), 8.02 (s, 1H), 8.38 (m, 1H), 10.28 (s, 1H).

### Example 20: Preparation of N-(3-methylcarbamoylphenyl)-4-methoxyphenylacetamide (Compound No. 60 in Table 1)

Melting point: 155-157°C
NMR (DMSO-d₆, δ): 2.75 (d, J=4.5Hz, 3H), 3.55 (s, 2H), 3.71 (s, 3H), 6.88 (d, J=8.8Hz, 2H), 7.24 (d, J=8.8Hz, 2H), 7.35 (m, 1H), 7.45 (d, J=7.8Hz, 1H), 7.74 (d, J=7.8Hz, 1H), 8.00 (s, 1H), 8.37 (m, 1H), 10.24 (s, 1H).

### Example 21: Preparation of N-(3-methylcarbamoylphenyl)-3,4-dimethoxyphenylacetamide (Compound No. 61 in Table 1)

NMR (DMSO-d₆, δ): 2.75 (d, J=4.5Hz, 3H), 3.55 (s, 2H), 3.71 (s, 3H), 3.73 (s, 3H), 6.82-6.94 (m, 3H), 7.35 (m, 1H), 7.46 (d, J=7.8Hz, 1H), 7.75 (d, J=7.8Hz, 1H), 8.00 (s, 1H), 8.37 (m, 1H), 10.22 (s, 1H).

### Example 22: Preparation of N-(3-methylcarbamoylphenyl)-3,5-dimethoxyphenylacetamide (Compound No. 62 in Table 1)

Melting point; 146-147°C
IR (KBr, cm⁻¹): 3341, 3246, 1667, 1638, 1589, 1547.
NMR (DMSO-d₆, δ): 2.76 (d, J=4.2Hz, 3H), 3.56 (s, 2H), 3.73 (s, 6H), 6.39 (s, 1H), 6.51 (s, 2H), 7.36 (dd, J=7.8Hz, 7.8Hz, 1H), 7.47 (d, J=7.8Hz, 1H), 7.75 (d, J=7.8Hz, 1H), 8.01 (s, 1H), 8.35 (d, J=4.2Hz, 1H), 10.23 (s, 1H).

### Example 23: Preparation of N-(3-methylcarbamoylphenyl)-3,4,5-trimethoxyphenylacetamide (Compound No. 63 in Table 1)

Melting point; 81-82°C
IR (KBr, cm⁻¹): 3304, 1642, 1589, 1554, 1508.
NMR (DMSO-d₆, δ): 2.76 (d, J=4.5Hz, 3H), 3.57 (s, 2H), 3.63 (s, 3H), 3.77 (s, 6H), 6.66 (s, 2H), 7.39 (dd, J=7.8Hz, 7.8Hz), 1H), 7.47 (d, J=7.8Hz, 1H), 7.76 (d, J=7.8Hz, 1H), 8.03 (s, 1H), 8.37 (d, J=4.5Hz, 1H), 10.23 (s, 1H).

### Example 24: Preparation of N-(3-methylcarbamoylphenyl)-3-benzyloxyphenylacetamide (Compound No. 68 in Table 1)

Melting point; 150°C
IR (KBr, cm⁻¹): 3302, 1661, 1634, 1586, 1530.
NMR (DMSO-d₆, δ): 2.76 (d, J=4.5Hz, 3H), 3.61 (s, 2H), 5.09 (s, 2H), 6.91 (dd, J=7.8Hz, 7.8Hz, 2H), 7.01 (s, 1H), 7.27 (dd, J=7.8Hz, 7.8Hz, 1H), 7.25-7.52 (m, 7H), 7.74 (d, J=7.8Hz, 1H), 8.02 (s, 1H), 8.36 (d, J=4.5Hz, 1H), 10.27 (s, 1H).

### Example 25: Preparation of N-(3-carbamoylphenyl)-3-hydroxyphenylacetamide (Compound No. 71 in Table 1)

Melting point; 188-189°C
NMR (DMSO-d₆, δ): 3.52 (s, 2H), 6.62 (m, 1H), 6.72-6.75 (m, 2H), 7.08 (m, 1H), 7.32-7.37 (m, 2H), 7.51 (d, J=6.9Hz, 1H), 7.76 (d, J=7.8Hz, 1H), 7.92 (s, 1H), 8.02 (s, 1H), 9.34 (s, 1H), 10.25 (s, 1H).

### Example 26: Preparation of N-(3-methylcarbamoylphenyl)-3-hydroxyphenylacetamide (Compound No. 72 in Table 1)

Melting point; 163°C
IR (KBr, cm⁻¹): 3333, 3293, 1676, 1640, 1588, 1562.
NMR (DMSO-d₆, δ): 2.74 (d, J=4.2Hz, 3H), 3.53 (s, 2H), 6.61 (d, J=7.2Hz, 1H), 6.72 (d, J=7.2Hz, 1H), 6.74 (s, 1H), 7.08 (dd, J=7.2Hz, 7.2Hz, 1H), 7.34 (dd, J=7.8Hz, 7.8Hz, 1H), 7.45 (d, J=7.8 Hz, 1H), 7.73 (d, J=7.8Hz), 1H), 7.80 (s, 1H), 8.34 (d, J=4.2Hz, 1H), 9.30 (s, 1H), 10.22 (s, 1H).

### Example 27: Preparation of N-(3-methylcarbamoylphenyl)-4-hydroxyphenylacetamide (Compound No. 73 in Table 1)

Melting point; 195-196°C
IR (KBr, cm⁻¹): 3393, 3283, 1661, 1638, 1541, 1518.
NMR (DMSO-d₆, δ): 2.73 (d, J=4.5Hz, 3H), 3.48 (s, 2H), 6.68 (d, J=8.4Hz, 2H), 7.10 (d, J=8.4Hz, 2H), 7.33 (dd, J=7.8Hz, 7.8Hz, 1H), 7.43 (d, J=8.7Hz, 1H), 7.72 (d, J=7.8Hz, 1H), 7.98 (s, 1H), 8.32 (d, J=4.5Hz, 1H), 9.20 (s, 1H), 10.14 (s, 1H).

### Example 28: Preparation of N-(3-methylcarbamoylphenyl)-3-nitrophenylacetamide (Compound No. 74 in Table 1)

Melting point: 139°C
IR (KBr, cm⁻¹): 3322, 3250, 1665, 1640, 1555, 1524.
NMR (DMSO-d₆, δ): 2.76 (d, J=4.5Hz, 3H), 3.86 (s, 2H), 7.37 (dd, J=7.8Hz, 7.8Hz, 1H), 7.49 (d, J=7.8Hz, 1H), 7.64 (dd, J=8.1Hz, 8.1Hz, 1H), 7.75 (d, J=8.1Hz, 1H), 7.80 (d, J=8.1Hz, 1H), 8.03 (s, 1H), 8.13 (d, J=8.1Hz, 1H), 8.24 (s, 1H), 8.37 (d, J=4.5Hz, 1H), 10.39 (s, 1H).

### Example 29: Preparation of N-(3-methylcarbamoylphenyl)-4-nitrophenylacetamide (Compound No. 75 in Table 1)

Melting point: 148-151°C
IR (KBr, cm⁻¹): 3277, 1663, 1640, 1588, 1522.
NMR (DMSO-d₆, δ): 2.74 (d, J=4.2Hz, 3H), 3.83 (s, 2H), 7.35 (dd, J=7.8Hz, 1H), 7.45 (m, 1H), 7.60 (d, J=8.7Hz, 2H), 7.72 (d, J=8.4Hz, 1H), 8.00 (s, 1H), 8.19 (d, J=8.7Hz, 2H), 8.33 (d, J=4.2Hz, 1H), 10.36 (s, 1H).

### Example 30: Preparation of N-(3-methylcarbamoylphenyl)-1-naphthylacetamide (Compound No. 113 in Table 1)

Melting point: 201-202°C
IR (KBr, cm⁻¹): 3274, 1657, 1640, 1588, 1532.
NMR (DMSO-d₆, δ): 2.75 (d, J=4.5Hz, 3H), 4.16 (s, 2H), 7.36 (dd, J=8.1Hz, 8.1Hz, 1H), 7.40-7.60 (m, 5H), 7.74 (d, J=7.8Hz, 1H), 7.84 (d, J=7.8Hz, 1H), 7.97 (d, J=7.8Hz, 1H), 8.03 (s, 1H), 8.35 (d, J=7.8Hz, 1H), 8.37 (d, J=4.5Hz, 1H), 10.44 (s, 1H).

### Example 31: Preparation of N-(3-methylcarbamoylphenyl)-2-naphthylacetamide (Compound No. 114 in Table 1)

Melting point: 175-176°C
IR (KBr, cm⁻¹): 3393, 1655, 1634, 1588, 1530.
NMR (DMSO-d₆, δ): 2.76 (d, J=4.5Hz, 3H), 3.83 (s, 2H), 7.37 (dd, J=7.8Hz, 7.8Hz, 1H), 7.40-7.55 (m, 4H), 7.77 (d, J=8.4Hz, 1H), 7.81-7.96 (m, 4H), 8.04 (s, 1H), 8.36 (d, J=4.5Hz, 1H), 10.37 (s, 1H).

### Example 32: Preparation of N-(3-methylcarbamoylphenyl)-3-indolylacetamide (Compound No. 140 in Table 1)

Melting point: 168-169°C
IR (KBr, cm⁻¹): 3382, 3287, 1655, 1636, 1588, 1555, 1528.
NMR (DMSO-d₆, δ): 2.73 (d, J=4.5Hz, 3H), 3.72 (s, 2H), 6.96 (dd, J=7.5Hz, 7.5Hz, 1H), 7.05 (dd, J=7.8Hz, 7.8Hz, 1H), 7.24 (s, 1H), 7.27-7.38 (m, 2H), 7.43 (d, J=7.8Hz, 1H), 7.59 (d, J=7.8Hz, 1H), 7.75 (d, J=8.7Hz, 1H), 8.00 (s, 1H), 8.32 (d, J=4.5Hz, 1H), 10.18 (s, 1H), 10.88 (s, 1H).

### Example 33: Preparation of N-(3-methylcarbamoylphenyl)-3-benzothienylacetamide (Compound No. 146 in Table 1)

Melting point: 194°C
IR (KBr, cm⁻¹): 3285, 1663, 1636, 1588, 1532.
NMR (DMSO-d₆, δ): 2.75 (d, J=4.2Hz, 3H), 3.94 (s, 2H), 7.32-7.53 (m, 4H), 7.61 (s, 1H), 7.76 (d, J=6.9Hz, 1H), 7.91 (d, J=7.2Hz, 1H), 7.98 (d, J=7.2Hz, 1H), 8.04 (s, 1H), 8.35 (d, J=4.2Hz, 1H), 10.40 (s, 1H).

### Example 34: Preparation of N-(3-methylcarbamoylphenyl)-4-benzothienylacetamide (Compound No. 148 in Table 1)

Melting point: 192°C
IR (KBr, cm⁻¹): 3295, 1676, 1632, 1595, 1559.
NMR (DMSO-d₆, δ): 2.76 (d, J=4.2Hz, 3H), 4.03 (s, 2H), 7.30-7.41 (m, 3H), 7.47 (d, J=7.8Hz, 1H), 7.65 (d, J=5.4Hz, 1H), 7.75 (d, J=6.3Hz, 1H), 7.77 (d, J=5.4Hz, 1H), 7.91 (m, 1H), 8.02 (s, 1H), 8.35 (d, J=4.2Hz, 1H), 10.39 (s, 1H).

### Example 35: Preparation of N-(3-methylcarbamoylphenyl)-2,2-dimethyl-2,3-dihydro-5-benzofuranylacetamide (Compound No. 157 in Table 1)

Melting point: 92-93°C
IR (KBr, cm⁻¹): 3289, 1665, 1611, 1589, 1555.
NMR (DMSO-d₆, δ): 1.39 (s, 6H), 1.53 (s, 6H), 2.75 (d, J=4.5Hz, 3H), 2.99 (s, 2H), 6.65 (d, J=8.4Hz, 1H), 7.05 (d, J=8.1Hz, 1H), 7.18 (s, 1H), 7.34 (dd, J=7.8Hz, 7.8Hz, 1H), 7.45 (d, J=7.5Hz, 1H), 7.80 (d, J=7.8Hz, 1H), 8.02 (s, 1H), 8.33 (d, J=4.5Hz, 1H), 9.18 (s, 1H).

### Example 36: Preparation of N-(3-methylcarbamoylphenyl)-3,4-methylenedioxyphenylacetamide (Compound No. 159 in Table 1)

Melting point: 174-175°C
IR (KBr, cm⁻¹): 3337, 3291, 1659, 1634, 1586, 1530, 1505.
NMR (DMSO-d₆, δ): 2.76 (d, J=4.5Hz, 3H), 3.55 (s, 2H), 5.98 (s, 2H), 6.74-6.93 (m, 3H), 7.36 (dd, J=7.8Hz, 7.8Hz, 1H), 7.47 (d, J=7.8Hz, 1H), 7.75 (d, J=7.8Hz, 1H), 8.01 (s, 1H), 8.35 (d, J=4.5Hz, 1H), 10.20 (s, 1H).

### Example 37: Preparation of N-(3-methylcarbamoylphenyl)phenoxyacetamide (Compound No. 176 in Table 1)

Melting point: 131°C
IR (KBr, cm⁻¹): 3378, 3283, 1669, 1640, 1588, 1535.
NMR (DMSO-d₆, δ): 2.75 (d, J=4.5Hz, 3H), 4.69 (s, 2H), 6.63-7.01 (m, 3H), 7.22-7.40 (m, 3H), 7.50 (d, J=7.8Hz, 1H), 7.77 (d, J=7.8Hz, 1H), 8.05 (s, 1H), 8.36 (d, J=4.5Hz, 1H), 10.18 (s, 1H).

### Example 38: Preparation of N-(3-methylcarbamoylphenyl)-2-chlorophenoxyacetamide (Compound No. 177 in Table 1)

Melting point: 172-173°C
IR (KBr, cm⁻¹): 3385, 3297, 1688, 1640, 1591, 1549.
NMR (DMSO-d₆, δ): 2.77 (d, J=4.5Hz, 3H), 4.85 (s, 2H), 6.99 (dd, J=7.5Hz, 7.5Hz, 1H), 7.08 (d, J=8.1Hz, 1H), 7.30 (dd, J=7.5Hz, 7.5Hz, 1H), 7.38-7.60 (m, 3H), 7.76 (d, J=8.4Hz, 1H), 8.06 (s, 1H), 8.42 (d, J=4.5Hz, 1H), 10.31 (s, 1H).

### Example 39: Preparation of N-(3-methylcarbamoylphenyl)-2-methylphenoxyacetamide (Compound No. 183 in Table 1)

Melting point: 148°C
IR (KBr, cm⁻¹): 3399, 3285, 1696, 1640, 1547.
NMR (DMSO-d₆, δ): 2.24 (s, 3H), 2.75 (d, J=4.5Hz, 3H), 4.70 (s, 2H), 6.80-6.90 (m, 2H), 7.07-7.19 (m, 2H), 7.37 (dd, J=8.1Hz, 7.8Hz, 1H), 7.49 (d, J=7.8Hz, 1H), 7.75 (d, J=8.1Hz, 1H), 8.05 (s, 1H), 8.35 (d, J=4.5Hz, 1H), 10.14 (s, 1H).

### Example 40: Preparation of N-(3-methylcarbamoylphenyl)-2-methoxyphenoxyacetamide (Compound No. 187 in Table 1)

Melting point: 133°C
IR (KBr, cm⁻¹): 3385, 3268, 1690, 1638, 1591, 1547.
NMR (DMSO-d₆, δ): 2.75 (d, J=4.5Hz, 3H), 3,79 (s, 3H), 4.66 (s, 2H), 6.82-7.02 (m, 4H), 7, 38 (dd, J=7.8Hz, 7.8Hz, 1H), 7.49 (d, J=7.8Hz, 1H), 7.75 (d, J=7.8Hz, 1H), 8.05 (s, 1H), 8.38 (d, J=4.5Hz, 1H), 10.18 (s, 1H).

### Example 41: Preparation of N-(3-methylcarbamoylphenyl)-1-naphthyloxyacetamide (Compound No. 191 in Table 1)

Melting point: 194°C
IR (KBr, cm⁻¹): 3405, 3304, 1696, 1638, 1541.
NMR (DMSO-d₆, δ): 2.75 (d, J=4.2Hz, 3H), 4.92 (s, 2H), 6.92 (d, J=7.8Hz, 1H), 7.33-7.62 (m, 6H), 7.79 (d, J=8.1Hz, 1H), 7.88 (m, 1H), 8.08 (s, 1H), 8.31 (m, 1H), 8.41 (d, J=4.2Hz, 1H), 10.36 (s, 1H).

### Example 42: Preparation of N-(3-methylcarbamoylphenyl)-2-naphthyloxyacetamide (Compound No. 192 in Table 1)

Melting point: 174°C
IR (KBr, cm⁻¹): 3382, 3275, 1672, 1638, 1588, 1557, 1534.
NMR (DMSO-d₆, δ): 2.75 (d, J=4.5Hz, 3H), 4.82 (s, 2H), 7.22-7.58 (m, 6H), 7.78-7.95 (m, 4H), 8.09 (s, 1H), 8.40 (d, J=4.5Hz, 1H), 10.28 (s, 1H).

### Example 43: Preparation of N-(3-methylcarbamoylphenyl)-2,3-dichlorophenoxyacetamide (Compound No. 204 in Table 1)

Melting point: 192-193°C
IR (KBr, cm⁻¹): 3385, 3291, 1692, 1644, 1547.
NMR (DMSO-d₆, δ): 2.77 (d, J=4.5Hz, 3H), 4.91 (s, 2H), 7.08 (d, J=8.1Hz, 1H), 7.20-7.45 (m, 3H), 7.52 (d, J=7.8Hz, 1H), 7.74 (d, J=8.7Hz, 1H), 8.05 (s, 1H), 8.42 (d, J=4.5Hz, 1H), 10.34 (s, 1H).

### Example 44: Preparation of N-(3-methylcarbamoylphenyl)-2-methyl-1-naphthylacetamide (Compound No. 216 in Table 1)

Melting point: 230-231°C
IR (KBr, cm⁻¹): 3299, 3071, 1684, 1638, 1589, 1560.
NMR (DMSO-d₆, δ): 2.50 (s, 3H), 2.73 (d, J=4.5Hz, 3H), 4.21 (s, 2H), 7.22-7.55 (m, 5H), 7.65-7.78 (m, 2H), 7.85 (d, J=7.8Hz, 1H), 8.01-8.15 (m, 2H), 8.36 (d, J=4.5Hz, 1H), 10.50 (s, 1H).

### Example 45: Preparation of N-(3-methylcarbamoylphenyl)-2-hydroxy-1-naphthylacetamide (Compound No. 219 in Table 1)

Melting point: 229-230°C
IR (KBr, cm⁻¹): 3310, 1686, 1613, 1582, 1561.
NMR (DMSO-d₆, δ): 2.75 (d, J=4.2Hz, 3H), 4.11 (s, 2H), 7.19 (d, J=9.0Hz, 1H), 7.20-7.50 (m, 4H), 7.66-7.82 (m, 3H), 7.87 (d, J=8.4Hz, 1H), 8.04 (s, 1H), 8.37 (d, J=4.5Hz, 1H), 9.79 (s, 1H), 10.32 (s, 1H).

### Example 46: Preparation of N-(3-methylcarbamoylphenyl)-3-phenylpropionamide (Compound No. 233 in Table 1)

Melting point: 142-143°C
IR (KBr, cm⁻¹): 3295, 1657, 1613, 1593, 1545.
NMR (DMSO-d₆, δ): 2.62 (t, J=7.8Hz, 2H), 2.75 (d, J=4.5Hz, 3H), 2.90 (t, J=7.8Hz, 2H), 7.10-7.40 (m, 6H), 7.44 (d, J=7.5Hz, 1H), 7.72 (d, J=7.5Hz, 1H), 7.99 (s, 1H), 8.33 (d, J=4.5Hz, 1H), 10.00 (s, 1H).

### Example 47: Preparation of N-(3-methylcarbamoylphenyl)-3-(2-methylphenyl)propionamide (Compound No. 240 in Table 1)

Melting point: 131°C
IR (KBr, cm⁻¹): 3289, 1674, 1640, 1555.
NMR (DMSO-d₆, δ): 2.29 (s, 3H), 2.57 (t, J=7.8Hz, 2H), 2.75 (d, J=4.2Hz, 3H), 2.88 (t, J=7.8Hz, 2H), 7.02-7.18 (m, 4H), 7.34 (dd, J=7.8Hz, 7.2Hz, 1H), 7.44 (d, J=7.2Hz, 1H), 7.73 (d, J=7.8Hz, 1H), 7.98 (s, 1H), 8.33 (d, J=4.2Hz, 1H), 10.01 (s, 1H).

### Example 48: Preparation of N-(3-methylcarbamoylphenyl)-3-(4-hydroxyphenyl)propionamide (Compound No. 245 in Table 1)

Melting point: 158°C
IR (KBr, cm⁻¹): 3424, 3285, 1647, 1553.
NMR (DMSO-d₆, δ): 2.54 (t, J=7.8Hz, 2H), 2.74 (d, J=4.2Hz, 3H), 2.78 (t, J=7.8Hz, 2H), 6.64 (d, J=8.1Hz, 2H), 7.01 (d, J=8.1Hz, 2H), 7.33 (dd, J=8.1Hz, 7.5Hz, 1H), 7.43 (d, J=7.5Hz, 1H), 7.71 (d, J=8.1Hz, 1H), 7.98 (s, 1H), 8.32 (d, J=4.2Hz, 1H), 9.10 (s, 1H), 9.97 (s, 1H).

### Example 49: Preparation of N-(3-methylcarbamoylphenyl)-3-(2-methoxyphenyl)propionamide (Compound No. 246 in Table 1)

Melting point: 150°C
IR (KBr, cm⁻¹): 3297, 1658, 1644, 1550.
NMR (DMSO-d₆, δ): 2.56 (t, J=7.2Hz, 2H), 2.75 (d, J=3.9Hz, 3H), 2.85 (t, J=7.2Hz, 2H), 3.78 (s, 3H), 6.84 (dd, J=7.5Hz, 7.5Hz, 1H), 6.93 (d, J=7.5Hz, 1H), 7.05-7.20 (m, 2H), 7.26 (dd, J=8.1Hz, 8.1Hz, 1H), 7.34 (d, J=8.1Hz, 1H), 7.72 (d, J=8.1Hz, 1H), 7.99 (s, 1H), 8.38 (d, J=3.9Hz, 1H), 9.67 (s, 1H).

### Example 50: Preparation of N-(3-methylcarbamoylphenyl)-3-(4-methoxyphenyl)propionamide (Compound No. 248 in Table 1)

Melting point: 151-152°C
IR (KBr, cm⁻¹): 3289, 1669, 1634, 1613, 1557, 1514.
NMR (DMSO-d₆, δ): 2.57 (t, J=7.5Hz, 2H), 2.77 (d, J=4.2Hz, 3H), 2.85 (t, J=7.5Hz, 2H), 3.71 (s, 3H), 6.84 (d, J=8.1Hz, 2H), 7.16 (d, J=8.1Hz, 2H), 7.35 (dd, J=7.8Hz, 7.8Hz, 1H), 7.45 (d, J=7.8Hz, 1H), 7.74 (d, J=7.5Hz, 1H), 8.01 (s, 1H), 8.34 (d, J=4.2Hz, 1H), 10.00 (s, 1H).

### Test Example 1: Measurement of anti-Helicobacter pylori activity

Brain heart infusion culture medium containing 10% fetal bovine serum (Difco) (5 ml) was taken in a test tube, and then the medium was inoculated with Helicobacter pylori strain 31A isolated from human (obtained from the Metropolitan Health Institute, Microorganism Department, First Laboratory of Bacteria). Cultivation was carried out under slightly aerobic condition (5% oxygen, 10% carbon dioxide, 85% nitrogen) at 37°C for 48 hours with shaking.

The culture was then inoculated to brain heart infusion medium containing 10% fetal bovine serum at a ratio of 5%, and added with a test compound dissolved in 10% dimethyl sulfoxide. Cultivation was carried out under slightly aerobic condition at 37°C for 48 hours with shaking, and then growth of Helicobacter pylori was examined. Antibacterial activity was recorded as the lowest concentration that exhibited growth inhibition (minimum inhibitory concentration: MIC). The results are shown in Table 2. From the results shown in Table 2, it can be understood that the compounds of the present invention have potent inhibitory activity against Helicobacter pylori.

**Table 2**

| Example No. (Compound No. in Table 1) | MIC (µg/ml) |
|---|---|
| 1 (No. 17) | 0.39 |
| 3 (No. 7) | 1.56 |
| 8 (No. 18) | 0.78 |
| 10 (No. 22) | 0.39 |
| 16 (No. 33) | 0.78 |
| 17 (No. 42) | 0.39 |
| 18 (No. 43) | 0.39 |
| 19 (No. 59) | 0.78 |
| 20 (No. 60) | 0.78 |
| 22 (No. 62) | 1.56 |
| 23 (No. 63) | 0.78 |
| 24 (No. 68) | 0.78 |
| 30 (No. 113) | 0.10 |
| 31 (No. 114) | 0.05 |
| 32 (No. 140) | 0.78 |
| 33 (No. 146) | 0.10 |
| 34 (No. 148) | 0.20 |
| 36 (No. 159) | 0.78 |
| 38 (No. 177) | 0.78 |
| 41 (No. 191) | 0.10 |
| 42 (No. 192) | 0.39 |
| 43 (No. 204) | 0.39 |
| 44 (No. 216) | 0.78 |
| 45 (No. 219) | 0.78 |
| 49 (No. 246) | 0.78 |

### Test Example 2: Measurement of anti-Campylobacter jejuni activity

According to a similar method to that of Test Example 1, inhibitory activity of the compound of the present invention against Campylobacter jejuni was determined. As a result, MIC of the compound as Example 31 was 0.008 µg/ml. From the result, it can be understood that the compound of the present invention has potent inhibitory activity against Campylobacter jejuni.

### Test Example 3: Acute toxicity test

The compound of the present invention, suspended in 0.5% CMC-Na aqueous solution, was forcibly administered orally to SD male and female rats, and symptoms of the rats were observed for seven days. As a result, each of LD₅₀ values of the compounds of Examples 30 and 31 was not lower than 2,000 mg/kg.

### Formulation Examples

### (1) Tablet

The following ingredients were mixed according to a conventional method, and compressed to obtain a tablet by using a conventional apparatus.

| | |
|---|---|
| Compound of Example 31 | 100 mg |
| Crystalline cellulose | 180 mg |
| Corn starch | 300 mg |
| Lactose | 600 mg |
| Magnesium stearate | 15 mg |

### (2) Soft capsule

The following ingredients were mixed according to a conventional method, and filled in a soft capsule.

| | |
|---|---|
| Compound of Example 41 | 100 mg |
| Olive oil | 900 mg |
| Lecithin | 60 mg |

### Industrial Applicability

The amide derivatives of the present invention have potent antibacterial activity against Helicobacter pylori, and therefore, they are useful as an active ingredient of medicaments.

## Claims

1. A compound represented by the following general formula (I) or a salt thereof, or a solvate thereof or a hydrate thereof: 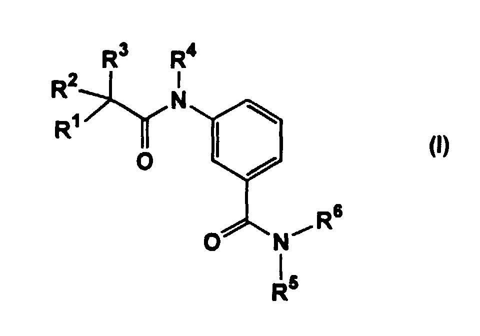 wherein R¹ represents a C₃-C₈ cycloalkyl group, a C₆-C₁₄ aryl group which may optionally be substituted, a residue of a heterocyclic compound which may optionally be substituted, a C₆-C₁₄ aryloxy group which may optionally be substituted, or a C₇-C₁₅ arylmethyl group which may optionally be substituted; R² and R³ independently represent hydrogen atom or a C₁-C₅ alkyl group, or R² and R³ may combine together to represent a C₂-C₇ alkylene group; R⁴ and R⁵ independently represent hydrogen atom or a C₁-C₅ alkyl group; and R⁶ represents hydrogen atom, a C₁-C₅ alkyl group which may optionally be substituted with a hydroxyl group, hydroxyl group, or a C₁-C₅ alkoxy group.

2. The compound according to claim 1 or a salt thereof, or a solvate thereof or a hydrate thereof, wherein R⁴ is hydrogen atom.

3. The compound according to claims 1 or 2 or a salt thereof, or a solvate thereof or a hydrate thereof, wherein R² and R³ are hydrogen atoms.

4. The compound according to any one of claims 1 to 3 or a salt thereof, or a solvate thereof or a hydrate thereof, wherein R⁵ is hydrogen atom.

5. The compound according to any one of claims 1 to 4 or a salt thereof, or a solvate thereof or a hydrate thereof, wherein R⁶ is a C₁-C₅ alkyl group.

6. The compound according to any one of claims 1 to 4 or a salt thereof, or a solvate thereof or a hydrate thereof, wherein R⁶ is methyl group.

7. The compound according to any one of claims 1 to 6 or a salt thereof, or a solvate thereof or a hydrate thereof, wherein R¹ is a C₆-C₁₄ aryl group which may optionally be substituted.

8. The compound according to any one of claims 1 to 6 or a salt thereof, or a solvate thereof or a hydrate thereof, wherein R¹ is a residue of a heterocyclic compound which may optionally be substituted.

9. The compound according to any one of claims 1 to 6 or a salt thereof, or a solvate thereof or a hydrate thereof, wherein R¹ is a C₆-C₁₄ aryloxy group which may optionally be substituted.

10. The compound according to any one of claims 1 to 6 or a salt thereof, or a solvate thereof or a hydrate thereof, wherein R¹ is a C₇-C₁₅ arylmethyl group which may optionally be substituted.

11. A compound selected from the group consisting of:
N-(3-methylcarbamoylphenyl)-3-chlorophenylacetamide;
N-(3-methylcarbamoylphenyl)-4-chlorophenylacetamide;
N-(3-methylcarbamoylphenyl)-3-bromophenylacetamide;
N-(3-methylcarbamoylphenyl)-4-bromophenylacetamide;
N-(3-methylcarbamoylphenyl)-3-methylphenylacetamide;
N-(3-methylcarbamoylphenyl)-4-methylphenylacetamide;
N-(3-methylcarbamoylphenyl)-3-methoxyphenylacetamide;
N-(3-methylcarbamoylphenyl)-4-methoxyphenylacetamide;
N-(3-methylcarbamoylphenyl)-3,4,5-trimethoxyphenylacetamide;
N-(3-methylcarbamoylphenyl)-3-benzyloxyphenylacetamide;
N-(3-methylcarbamoylphenyl)-1-naphthylacetamide;
N-(3-methylcarbamoylphenyl)-2-naphthylacetamide;
N-(3-methylcarbamoylphenyl)-3-indolylacetamide;
N-(3-methylcarbamoylphenyl)-3-benzothienylacetamide;
N-(3-methylcarbamoylphenyl)-4-benzothienylacetamide;
N-(3-methylcarbamoylphenyl)-3,4-methylenedioxyphenylacetamide;
N-(3-methylcarbamoylphenyl)-2-chlorophenoxyacetamide;
N-(3-methylcarbamoylphenyl)-2,3-dichlorophenoxyacetamide;
N-(3-methylcarbamolyphenyl)-1-naphthyloxyacetamide;
N-(3-methylcarbamoylphenyl)-2-naphthyloxyacetamide; and
N-(3-methylcarbamoylphenyl)-3-(2-methoxyphenyl)propionamide,
or a salt thereof, or a solvate thereof or a hydrate thereof.

12. N-(3-methylcarbamoylphenyl)-1-naphthylacetamide or a salt thereof, or a solvate thereof or a hydrate thereof.

13. N-(3-methylcarbamoylphenyl)-2-naphthylacetamide or a salt thereof, or a solvate thereof or a hydrate thereof.

14. N-(3-methylcarbamoylphenyl)-3-benzothienylacetamide or a salt thereof, or a solvate thereof or a hydrate thereof.

15. N-(3-methylcarbamoylphenyl)-4-benzothienylacetamide or a salt thereof, or a solvate thereof or a hydrate thereof.

16. N-(3-methylcarbamoylphenyl)-1-naphthyloxyacetamide or a salt thereof, or a solvate thereof or a hydrate thereof.

17. A medicament comprising as an active ingredient a substance selected from the group consisting of a compound according to any one of claims 1 to 16 and a salt thereof, and a solvate thereof and a hydrate thereof.

18. The medicament according to claim 17 which is in the form of a pharmaceutical composition comprising said substance as an active ingredient and one or more pharmaceutical additives.

19. The medicament according to claims 17 or 18 which has antibacterial activity.

20. The medicament according to claim 19 which has antibacterial activity against a microorganism belonging to the genus Helicobacter and/or Campylobacter.

21. The medicament according to claim 20 which has anti-Helicobacter pylori activity and/or anti-Campylobacter jejuni activity.

22. The medicament according to any one of claims 17 to 21 which is used for preventive and/or therapeutic treatment of a digestive disease.

23. The medicament according to claim 22 wherein the digestive disease is selected from the group consisting of gastritis, gastric ulcer, gastric cancer, gastric malignant lymphoma, MALT lymphoma, duodenal ulcer, duodenal carcinoma, and enteritis.

24. The medicament according to any one of claims 17 to 21 which is used for preventive treatment of recurrence of a digestive disease.

25. The medicament according to claim 24 wherein the digestive disease is gastric ulcer or duodenal ulcer.

26. A use of a substance selected from the group consisting of a compound according to any one of claims 1 to 16 and a salt thereof, and a solvate thereof and a hydrate thereof for a manufacture of a medicament according to any one of claims 17 to 25.
